# EUROPEAN PATENT APPLICATION

(11) **EP 3 900 707 A1**
(43) Date of publication of application: **27.10.2021**
(21) Application number: 20171441.7
(22) Date of filing: 24.04.2020
(51) Int. Cl.: A61K 9/16, A61K 9/48, A61P 37/06, A61K 31/4439, C07D 401/12, A61P 1/16, A61P 13/10, A61P 13/12

(54) **SYSTEMIC FORMULATION OF A PYRIDINONE DERIVATE FOR TG2-RELATED DISEASES**

(71) Applicant: Dr. Falk Pharma GmbH, 79108 Freiburg (DE); Zedira GmbH, 64293 Darmstadt (DE)
(72) Inventor: GREINWALD, Roland, 79341 Kenzingen (DE); HILS, Martin, 64295 Darmstadt (DE); MOHR, Wolfgang, 79106 Freiburg (DE); PASTERNACK, Ralf, 64347 Griesheim (DE); TEWES, Bernhard, 79279 Vörstetten (DE); WILHELM, Rudolf, 76476 Bischweier (DE)
(74) Representative: Arth, Hans-Lothar

(57) **Abstract**

The present invention relates to a formulation in particular an oral formulation for the prophylaxis and treatment of TG2-related disorders like fibrosis in particular diabetic nephropathy and/or diabetic associated non-alcoholic steatohepatitis (NASH) and/or non-alcoholic steatohepatitis, and its use in the prophylaxis and/or treatment of fibrosis in particular nephropathy, NASH, idiopathic pulmonary fibrosis, and cystic fibrosis.

## Description

The present invention relates to a systemic formulation in particular an oral formulation for the prophylaxis and/or treatment TG2-related diseases such as fibrosis in particular nephropathy, fibrotic liver diseases including non-alcoholic fatty liver disease (NAFLD) and non-alcoholic steatohepatitis (NASH), idiopathic pulmonary fibrosis (IPF) and cystic fibrosis, and its use in the prophylaxis and/or treatment of fibrosis in particular nephropathy, fibrotic liver diseases including NAFLD and NASH, idiopathic pulmonary fibrosis and cystic fibrosis.

### Background of the invention

Steatohepatitis is a type of fatty liver disease, characterized by inflammation of the liver with concurrent fat accumulation in hepatocytes. Mere deposition of fat in the liver is termed steatosis, and together these constitute fatty liver changes.

There are two main types of fatty liver disease: alcohol-related fatty liver disease and non-alcoholic fatty liver disease (NAFLD). Risk factors for NAFLD include diabetes, obesity and metabolic syndrome. When inflammation is present it is referred to as alcoholic steatohepatitis and non-alcoholic steatohepatitis (NASH). Untreated steatohepatitis of either may cause progress to fibrosis and subsequent cirrhosis, and NASH is now believed to be a frequent cause of unexplained cirrhosis.

Diabetic nephropathy is a kidney disease that develops as a result of diabetes mellitus (DM). Diabetes mellitus, and Type 2 Diabetes Mellitus (T2DM) is the most common cause of end stage renal disease (ESRD). Diabetic nephropathy generally results in a chronic and progressive degradation of kidney function, to the point where the patient must undergo dialysis or receive a transplant to survive. The initial stage of subtle morphologic changes in the renal glomeruli is followed by microalbuminuria. This is associated with a modestly rising blood pressure and an increased incidence of cardiovascular disease. There follows a continued increase in urinary protein excretion and defining glomerular filtration rate. Diabetic nephropathy has many possible underlying pathophysiological causes including metabolic, glycosylation of proteins, haemodynamics, altered flow/pressure in glomeruli, the development of hypertension and cytokine production; all of these are associated with the development of extracellular matrix and increased vascular permeability leading to glomerular damage and proteinuria.

Huang et al. (Kidney International 2009, 76, 383 - 394) describe a dipeptide-derivative (NTU281) as an **irreversible** TG2-Inhibitor. One of the drawbacks of said dipeptide-derivative is that the dipeptide-derivative has to be applied by an implanted osmotic pump locally into the kidney. A systemic formulation of the dipeptide-derivative according to Johnson is not conceivable on the basis of this teaching since TG2 is ubiquitously expressed in almost all cell types and cell compartments, is present on the cell surface and gets secreted to the extracellular matrix, and is present in various organs, so that by applying a TG2-inhibitor unwanted off-target-effects was most likely.

Huang et al. describe an implanted osmotic pump containing a topical formulation NTU281 (drug) in phosphate-buffered saline as a vehicle (50 mmol/l). Thereby, the osmotic pump has to be implanted by a surgical intervention being connected with anaesthesia. Although anaesthesia is commonly used in the medical field, a significant risk for complications is always associated, in particular when vulnerable populations as children and elderly are treated. The implantation of an osmotic pump into the body of a patient cause an additional risk for the patient since electronic devices like pumps are sensitive under physiological conditions and might for example burst, and it is thus not always reliable. Moreover, the pump has to be maintained which causes an additional suffering for the patient. Furthermore, the patient compliance can be improved remarkably since the obstacle of surgical intervention is removed. An implanted osmotic pump containing a topical formulation cannot be used in human.

Lauzier et al. (Arthritis Research Therapy 2012, 14, R159) describe the influence cystamine or a RNA-derivative for the inhibition of TG2 by cystamine, a competitive inhibitor of TGase on the invadopodia formation and cartilage breakdown in arthritis. Cystamine is a disulfide having two amino moieties.

Luciani et al. (Nature Cell Biology 2010, 12, 863-875) describe the influence of cystamine and siRNA on the lung inflammation in cystic fibrosis.

Luo et al. (Journal of the American Heart Association, 2016, 1-12) disclose the influence of 1,3-dimethyl-2-[(2-oxopropyl)-thio]-imidazolium (R283) or halo-dihydroisoxazole-derivate transglutaminase inhibitor (KCC009) on inflammation in cystic fibrosis.

Olsen et al. (American Journal of Respiratory Cell and Molecular Biology 2014, 50, 737-747) disclose the influence of two different small electrophilic compounds, 2-cyano-3,12-dioxoolean-1,9-dien-28-oic acid and 15-deoxydelta- 12,14-prostaglandin J2 on pulmonary fibrosis.

Sanchez-Lara et al. (Veterinary Pathology 2015, Vol. 52(3) 513-523) describe the influence of 1,3-dimethyl-2[(oxopropyl)thio]-imidazolium chloride (D003, Zedira), the nonselective TG inhibitor NTU281, or monoclonal antibody BB7 on chronic kidney disease.

It is still a great problem in the pharmaceutical field to establish an appropriate bioavailability of a drug under physiological conditions in particular in case of a systemic therapy.

It is the objective of the present invention to provide means for the treatment of diabetic nephropathy and/or non-alcoholic steatohepatitis exhibiting a bioavailability, and a high anti-fibrotic effect along with low side-effects.

The objective of the present invention is solved by the teaching of the independent claims. Further advantageous features, aspects and details of the invention are evident from the dependent claims, the description, the figures, and the examples of the present application.

### Brief description of the invention

The objective of the present invention is solved by a formulation preferably a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate of the formula (I): 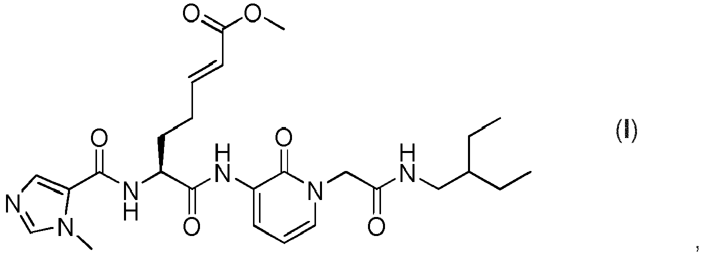 or an enantiomer, a solvate, hydrate or a pharmaceutically acceptable salt of formula (**I**).

Unexpectedly, it could be found that a compound according to formula (**I**) can be used to reduce fibrosis in particular fibrosis caused by a diabetic nephropathy and non-alcoholic steatohepatitis. The compound contains a pyridinone moiety as a main structural element. Further, it is surprising that a compound according to formula (**I**) can be used in the prophylaxis and treatment of nephropathy in particular diabetic nephropathy, liver fibrosis, and cystic fibrosis as a systemic formulation, i.e. the drug is distributed through the blood or lymphatic system throughout the body. This is particularly surprising due to the fact that TG2 is ubiquitously expressed in almost all cell types and cell compartments, is present on the cell surface and gets secreted to the extracellular matrix, and is present in various organs, and thus it could be envisioned that off-target effects would be most likely. The NASH- and mouse model study confirms the anti-fibrotic effect on the liver and kidney being achieved by the administration of a systemic formulation containing the compound according to formula (**I**) (example 2 and 3). Moreover, positive data of the bioavailability study in mouse (example 4) also confirms the bioavailability of compound (I) by a systemic administration *in vivo.* Suprisingly, it was found that the bioavailability of compound (I) could be largely increased in human by application of the systemic formulation (example 7).

In addition, a systemic formulation avoids means like an osmotic pump such as used by Johnson et al., and an implantation of said device can be completely avoided, and thus the administration of the drug is objectively facilitated, more reliable, safer, and the suffering for the patient can be significantly and objectively be reduced. The formulation according to the invention can be administered much easier than the formulation of the state of the art. In addition, the formulation according to the invention exhibits a higher anti-fibrotic effect, and thus a better result of the therapy.

Diabetes is also often associated with NASH (non-alcoholic steatohepatitis) which is a frequent cause of unexplained cirrhosis. It is apparent that the treatment of NASH and a diabetic nephropathy is often required at the same time. A systemic formulation according to the invention is usable to target the liver (NASH) and the kidneys (diabetic nephropathy) simultaneously. Likewise, cholestatic liver diseases, such as PSC (primary sclerosing cholangitis) and PBC (primary biliary cholangitis) are fibrotic liver diseases which are often associated with cholemic nephropathy, a chronic and fibrotic inflammation of the kidney caused by high exposure to endogenous bile acids. Also possible is the treatment of the kidneys, and prophylactic treatment of the liver at the same time, and vice versa. Thus, apparently the systemic formulation according to the invention enables its use in a new clinical situation. In addition, cystic fibrosis is often related to diabetes. It could be shown that the a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate exhibit the same anti-fibrotic effect in the liver and in the kidney (Example 5).

### Description of the invention

The term **"systemic formulation"** refers to a pharmaceutical composition suitable for administration such that a drug or active agent is administered systemically throughout the body of an organism, e.g. a form of medication for the circulatory system so that the entire body is affected. Administration can take place via enteral administration (absorption of the drug through the gastrointestinal tract) or parenteral administration (e.g. pulmonary, nasal, injection or infusion). Preferably, the term "systemic formulation" excludes formulation for an intravenous application. The **circulatory system**, also called the **cardiovascular system** or the **vascular system**, is an organ system that permits blood to circulate and transport nutrients (such as amino acids and electrolytes), oxygen, carbon dioxide, hormones, and blood cells to and from the cells in the body to provide nourishment and help in fighting diseases, stabilize temperature and pH, and maintain homeostasis.
The circulatory system includes the lymphatic system, which circulates lymph. The passage of lymph for example takes much longer than that of blood. Thus, the term "systemic formulation" refers to a formulation, wherein the drug is distributed throughout the body of an organism by e.g. the blood or lymphatic system throughout the body, for example, after an intravenous or intramuscular injection or taking a tablet, i.e. after an enteral, in particular an oral or a parenteral administration.
The systemic formulations as disclosed herein are preferably in the form of a tablet, capsule, powder, or granules.

In contrast thereto, a "**topical formulation**" is a formulation that is applied to a particular place on or in the body where it should act. Topical means "place", "locally", at a specific site", "externally" or "limited to a specific site of the body".

Thus, the risk of possible unwanted side effects in other areas of the organism can be reduced. Most often topical administration means application to body surfaces such as the skin or mucous membranes to treat ailments via a large range of classes including creams, foams, gels, lotions, and ointments. Many topical medications are epicutaneous, meaning that they are applied directly to the skin. Topical medications may also be inhalational, such as asthma medications, or applied to the surface of tissues other than the skin, such as eye drops applied to the conjunctiva, or ear drops placed in the ear, medications applied to the surface of a tooth or application of the drug by means of a pump such as an osmotic pump.
The topical formulations include aural, buccal, endobronchial, epicutaneous, inhalation, intraarticular, into the gluteus maximus muscle, intracardiac, intracutaneous, intralumbar, intralymphatic, intramammarial, intranasal, intraneuronal, intraocular, intraorbital, intraosseous, intrapericadial, intrapulmonary, intrathecal, intratracheal, intraurethral, intrauterine, intraventricular, intravesical, intravitreal, conjunctival, cutan, nasal, perineural, retrobulbar, subconjunctival, vaginal, and ciliary.

The term **"parenteral formulation",** as used herein refers, to a formulation, which usually is administered by injection or infusion, and includes, without limitation, epidural, intraarterial, intravenous, intravasal, intravascular, intramuscular, intraperitoneal, intrapleural, subcutaneous, subcuticular, and transdermal injection and infusion. Preferably, a parenteral formulation is selected from the group comprising or consisting of epidural, intravasal, intravascular, intramuscular, intraperitoneal, intrapleural, subcutaneous, subcuticular, and transdermal injection and infusion. Preferably, the intraarterial and intravenous formulations are excluded from the parenteral formulations.

**"Enteral formulation"**, as used herein, refers to a formulation being usually a medication which is absorbed through the mouth (per os, orally, perorally): tablets, dragees, capsules, juices, drops, etc. These medicines are absorbed into the blood in the gastrointestinal tract, and then enter the liver via the portal vein system and then into the bloodstream via the hepatic vein. The term, as used herein, refers to a formulation which is usually administered including, without limitation enteral, intragastral, sublingual, peroral (oral), and rectal. Preferably, enteral formulation consists of a formulation selected from the group comprising or consisting of enteral, intragastral, sublingual, peroral (oral), and rectal.

**"Oral formulation",** as used herein, refers to a formulation being a medication which is absorbed through the mouth (per os, orally, perorally tablets, dragees, capsules, juices, drops, etc.). These medicines are absorbed into the blood in the gastrointestinal tract, and then enter the liver via the portal vein system and then into the bloodstream via the hepatic vein. The term, as used herein, refers to a formulation which is administered orally.

The systemic formulation can be in a liquid or solid form including solutions, oral drops, suspensions, emulsions, powders and granules such as effervescent granules, tablets such as uncoated tablets, coated tablets, effervescent tablets, soluble tablets, chewable tablets, oral lyophilisates, lozenges, pastilles, compressed lozenges, sublingual tablets, buccal tablets, granules, effervescent granules and capsules. In particular, the systemic formulation can be a liquid preparation including oral solutions, suspensions, emulsions, powders and granules for oral solutions and suspensions, oral drops, powder for oral drops, syrups and powder and granules for syrups or in a solid form including uncoated tablets, coated tablets, effervescent tablets, soluble tablets, chewable tablets, oral lyophilisates, lozenges, pastilles, compressed lozenges, sublingual tablets, buccal tablets, granules, effervescent granules and capsules. Uncoated and coated tablets, and capsules, either hard or soft are the preferred pharmaceutical formulations. Most preferably, the formulation is a tablet or a capsule. As an example, there may be mentioned water or water/propylene glycol solutions for parenteral injections or addition of sweeteners and opacifiers for oral solutions, suspensions, and emulsions. Preferably, the systemic formulation is a solid formulation, more preferably a solid enteral formulation, and most preferably a solid oral formulation.

"Topical administration", as used herein, refers to the administration of a topical formulation.

"Systemic administration", as used herein, refers to the administration of a systemic formulation.

"Topical availability", as used herein, refers to the release of the drug from its formulation such as from a vehicle of a formulation or from a tablet to the place at which the drug should be absorbed by the specific tissue or organ so that the drug could act at all.

"Systemic availability", as used herein, refers to the proportion of the dose of a drug that reaches the systemic circulation intact after administration by a route other than intravenous. The term "systemic availability" also refers to the extent to which a drug or other substance is taken up by a specific tissue or organ after administration. For example, a drug which is orally administered and overcomes the epithelium barrier of the intestine is in the tissue of the intestine, and thus it has a systemic availability or in the other words it is systemic available. "Systemic availability" and "systemic available" are synonymous for "bioavailability" or "bioavailable". Thus, also topically administered compounds can exhibit a systemic availability.

The term "drug level" refers to the level of the drug in the plasma, tissue or organ, and the phrase "systemic availability at the target site" refers to the same aspect.

As used herein, the term "pharmaceutically acceptable salts" refers to salts of certain ingredient(s) which possess the same activity as the unmodified compound(s) and which are neither biologically nor otherwise undesirable.
A pharmaceutically acceptable salt can be formed with, for example, organic or inorganic acids. Suitable acids include acetic acid, acetylsalicylic acid, organic di-carboxylic acid such as oxalic acid, malonic acid, succinic acid, glutaric acid, tartaric acid, fumaric acid, maleic acid, malic acid, adipic acid, or glutamic acid, and organic tri-carboxylic acid such as citric acid, or sodium hydrogen citrate alginic acid, ascorbic acid, aspartic acid, benzoic acid, benzenesulfonic acid, bisulfic acid, boric acid, butyric acid, camphoric acid, camphorsulfonic acid, carbonic acid, citric acid, cyclopentanepropionic acid, digluconic acid, dodecylsulfic acid, ethanesulfonic acid, formic acid, fumaric acid, glyceric acid, glycerophosphoric acid, glycine, glucoheptanoic acid, gluconic acid, glutamic acid, glutaric acid, glycolic acid, hemisulfic acid, heptanoic acid, hexanoic acid, hippuric acid, hydrobromic acid, hydrochloric acid, hydroiodic acid, hydroxyethanesulfonic acid, lactic acid, maleic acid, malic acid, malonic acid, mandelic acid, methanesulfonic acid, mucic acid, naphthylanesulfonic acid, naphthylic acid, nicotinic acid, nitrous acid, oxalic acid, pelargonic, phosphoric acid, propionic acid, saccharin, salicylic acid, sorbic acid, succinic acid, sulfuric acid, tartaric acid, thiocyanic acid, thioglycolic acid, thiosulfuric acid, tosylic acid, undecylenic acid, and naturally and synthetically derived amino acids. Preferably, the acid is adipic acid.

Thus, a preferred embodiment of the present invention is directed to the salt of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate of the formula (**I**): 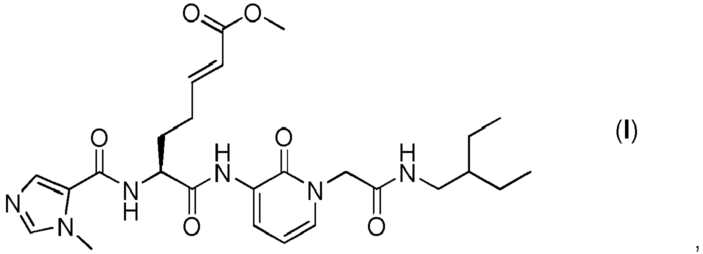 and adipic acid.

Another preferred embodiment of the invention is related to a systemic formulation containing a salt of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate of the formula (**I**): 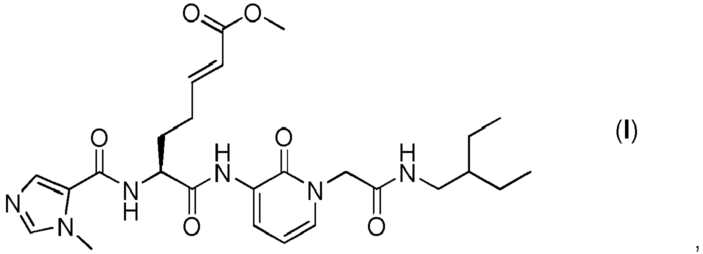 and adipic acid, or an enantiomer, a solvate or a hydrate of the salt of formula (**I**) and adipic acid.

As used herein, the term "solvates" refers to those forms of a compound in particular the (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate which form a complex through coordination with solvent molecules.

As used herein, the term "hydrates" refers to those forms of a compound in particular the (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate which form a complex through coordination with water molecules.

As used herein, the term "effective amount" or "therapeutically effective amount" of an active agent or a pharmaceutically active agent or a drug or an active pharmaceutical ingredient, which are synonymous herein, refers to an amount of the active agent or pharmaceutically active agent or drug or active pharmaceutical ingredient, sufficient enough to have a positive effect. Accordingly, these amounts are disease to be treated but low enough to avoid serious side effects. A therapeutically effective amount of the pharmaceutically active agent will cause a substantial relief of symptoms when applied repeatedly over time. Effective amounts of the pharmaceutically active agent will vary with the particular condition or conditions being treated, the severity of the condition, the duration of the treatment, the specific components of the composition being used, and like factors.

As used herein, the term "active agent", "pharmaceutically active agent", "drug" or "active pharmaceutical ingredient", which are synonymously used herein, refers to a compound exhibiting a therapeutic effect upon a mammal in particular a human.

As used herein, the term "pharmaceutical composition" refers to a composition which, upon administration, demonstrates a therapeutic effect upon a mammal.

Systemic formulations according to the invention as described herein, preferably contains (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate.

An embodiment of the present invention is thus direct to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate of the formula (**I**):

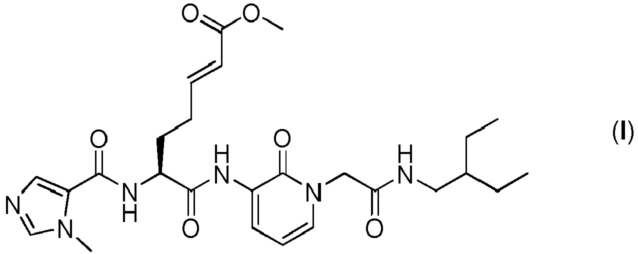

Further, the systemic formulation can be an enteral or parenteral formulation. An embodiment according to the invention is therefore related to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, wherein the systemic formulation is in form of an enteral or parenteral formulation.

It is preferred if the systemic formulation is in form of an oral formulation. An oral formulation is a specific form of an enteral formulation. Therefore, a preferred embodiment of the invention is directed to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, wherein the systemic formulation is in form of an oral formulation.
Furthermore, (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate can be administered in form of its pharmaceutically active salts, solvate or hydrate, optionally using essentially non-toxic pharmaceutically acceptable excipients. Formulations are prepared in a known manner in a conventional solid or fluid carrier using conventional pharmaceutically acceptable excipients in a suitable dose.

Thus, the systemic formulation according to the invention can further comprise an excipient. An embodiment according to the invention is thus directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient.

After the administration of the systemic formulation, the administered drug dose has to dissolve quickly and completely. The pH variations in the stomach after the oral administration have to be regulated and it should be ensured that the administered drug dose is dissolved. The bigger the systemic availability (AUC) in conjunction with the mucosal release in the small intestine, the higher pharmacological effect to be expected.
The excipient can be an acidifier. The term "acidifier" refers to a substance which, when dissolved in water, produces a pH level of less than 7.0. Thus, systemic formulations according to the invention can comprise an acidifier. Acidifiers include organic acids such as ascorbic acid, organic di-carboxylic acid such as oxalic acid, malonic acid, succinic acid, glutaric acid, tartaric acid, fumaric acid, maleic acid, malic acid, adipic acid (hexanedioic acid), or glutamic acid, and organic tri-carboxylic acid such as citric acid, or sodium hydrogen citrate. Preferably, the acidifier is adipic acid.
Preferred "acidifiers" for the systemic formulations as disclosed herein are selected from the group consisting of ascorbic acid, organic di-carboxylic acids such as oxalic acid, malonic acid, succinic acid, glutaric acid, tartaric acid, fumaric acid, maleic acid, malic acid, adipic acid, glutamic acid, and organic tri-carboxylic acids, citric acid, and sodium hydrogen citrate.

Thus, the systemic formulation according to the invention can comprise an acidifier.

A preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is an acidifier.

Another preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one acidifier.

A preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is an acidifier, and wherein at least one acidifier is selected from the group comprising or consisting of organic di-carboxylic acid and organic tri-carboxylic acid. Preferably, at least one acidifier is selected from the group consisting of organic di-carboxylic acid and organic tri-carboxylic acid. More preferably, the at least one acidifier is selected from the group the group comprising or consisting of organic di-carboxylic acid and organic tri-carboxylic acid. Even more preferably, the at least one acidifier is selected from the group the group consisting of organic di-carboxylic acid and organic tri-carboxylic acid.

Thus, a preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one acidifier, wherein at least one acidifier is selected from the group comprising or consisting of organic di-carboxylic acid and organic tri-carboxylic acid.

A preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is an acidifier, and wherein at least one acidifier is selected from the group comprising or consisting of oxalic acid, malonic acid, succinic acid, glutaric acid, tartaric acid, fumaric acid, maleic acid, malic acid, adipic acid (hexanedioic acid), or glutamic acid. Preferably, at least one acidifier is selected from the group consisting of oxalic acid, malonic acid, succinic acid, glutaric acid, tartaric acid, fumaric acid, maleic acid, malic acid, adipic acid (hexanedioic acid), or glutamic acid. More preferably, the at least one acidifier is selected from the group comprising or consisting of oxalic acid, malonic acid, succinic acid, glutaric acid, tartaric acid, fumaric acid, maleic acid, malic acid, adipic acid (hexanedioic acid), or glutamic acid. Even more preferably, the at least one acidifier is selected from the group consisting of oxalic acid, malonic acid, succinic acid, glutaric acid, tartaric acid, fumaric acid, maleic acid, malic acid, adipic acid (hexanedioic acid), or glutamic acid.

A preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one acidifier, wherein at least one acidifier is selected from the group comprising or consisting of oxalic acid, malonic acid, succinic acid, glutaric acid, tartaric acid, fumaric acid, maleic acid, malic acid, adipic acid (hexanedioic acid), or glutamic acid.

A particularly preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate, and at least one excipient or an enantiomer, a solvate, hydrate or a pharmaceutically acceptable salt thereof, wherein at least one excipient is an acidifier, wherein the acidifier is adipic acid.

A particularly preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one acidifier, wherein at least one acidifier is adipic acid.

A particularly preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and adipic acid.

The drug solution is transported to the small intestine after the stomach passage. This passage is - at least in the fasted state - connected with an increase of the pH from about 2 to about 6. The drug dose has to remain in the solution, i.e. the drug should not precipitate. This effect can be achieved by the addition of a polymeric precipitation inhibitor. Thus, the polymeric precipitation inhibitor inhibits the crystallization of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and functions as a crystallization inhibitor.

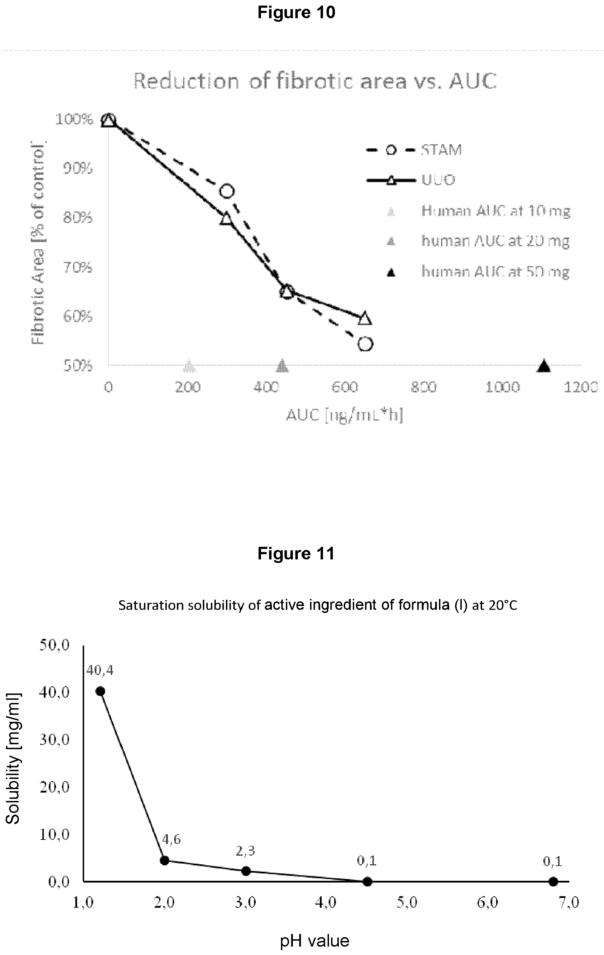

The saturation solubility of said compound is low at a pH value in the small intestine (example 10 and Figure 11). The solution is stabilized by the addition of polymeric precipitation inhibitor which can also act as a binder. When the drug is exposed to an aqueous medium, the polymeric precipitation inhibitor decelerates the precipitation of the drug preferably by complexation. Apart from that, the polymeric precipitation inhibitor increases the viscosity in the medium which further intensifies the effect.
Thus, the systemic formulation according to the invention can further comprise a polymeric precipitation inhibitor. Thus, the excipient can be a polymeric precipitation inhibitor. The term "polymeric precipitation inhibitor" refers to a material that decelerates the precipitation of a drug. Suitable polymeric precipitation inhibitor includes L-hydroxypropyl cellulose, hydroxypropyl cellulose, a combination of L-hydroxypropyl cellulose and hydroxypropyl cellulose, polyethylene glycol (PEG), poly(ethylene oxide)-poly (propylene oxide)-poly (ethylene oxide) (= poloxamer), polyvinyl alcohol (PVA), polyvinylpyrrolidone (PVP), carboxymethyl cellulose (CMC), methylcellulose (MC), hydroxyethylcellulose (HEC), hydroxypropylmethylcellulose (HPMC), ethylcellulose (EC), and/or sodium carboxymethyl cellulose. Preferably, the polymeric precipitation inhibitor is selected from the group comprising or consisting of cellulose and cellulose derivative. More preferably, the polymeric precipitation inhibitor is a cellulose, a cellulose derivative, a combination of cellulose and a cellulose derivative, or a combination of cellulose and a cellulose derivative. Even more preferably, the polymeric precipitation inhibitor is selected from the group comprising or consisting of L-hydroxypropyl cellulose and hydroxypropyl cellulose. Most preferably, the polymeric precipitation inhibitor is L-hydroxypropyl cellulose, hydroxypropyl cellulose, or a combination of L-hydroxypropyl cellulose and hydroxypropyl cellulose. The combination of L-hydroxypropyl cellulose and hydroxypropyl cellulose acts as polymeric precipitation inhibitor and disintegrant so that the amount of the disintegrant can be reduced.
The systemic formulations as disclosed herein and especially the systemic formulations for oral administration contain as polymeric precipitation inhibitor most preferably L-hydroxypropyl cellulose, hydroxypropyl cellulose or a combination of L-hydroxypropyl cellulose and hydroxypropyl cellulose.

The systemic formulations as disclosed herein and especially the systemic formulations for oral administration comprise most preferably at least one acidifier and/or at least one polymeric precipitation inhibitor.

The systemic formulations as disclosed herein and especially the systemic formulations for oral administration contain most preferably adipic acid as acidifier and L-hydroxypropyl cellulose, hydroxypropyl cellulose or a combination of L-hydroxypropyl cellulose and hydroxypropyl cellulose as polymeric precipitation inhibitor.

An embodiment of the invention is thus related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a polymeric precipitation inhibitor.

An embodiment of the invention is thus related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one polymeric precipitation inhibitor.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is polymeric precipitation inhibitor, and wherein the polymeric precipitation inhibitor is selected from the group comprising or consisting of cellulose and cellulose derivative.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one polymeric precipitation inhibitor selected from the group comprising or consisting of cellulose and cellulose derivative.

An embodiment of the invention is thus related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a polymeric precipitation inhibitor, and wherein the polymeric precipitation inhibitor is L-hydroxypropyl cellulose and/or hydroxypropyl cellulose.

An embodiment of the invention is thus related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one polymeric precipitation inhibitor selected from the group comprising or consisting of L-hydroxypropyl cellulose and/or hydroxypropyl cellulose.

An embodiment of the invention is thus related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and L-hydroxypropyl cellulose and/or hydroxypropyl cellulose.

The systemic formulation according to the invention can comprise a binder. Thus, the excipient can be a binder. Binders are characterized as substances binding or "gluing" powders to each other and they consequently serve as "glue" in the formulation. In other words, a binder is a material that holds or draws other materials together to form a cohesive whole mechanically, chemically, by adhesion or cohesion. Suitable binders include sugar, such as sucrose; polysaccharides such as xanthan gum, guar gum, carrageenan, starches derived from wheat, corn, rice and potatoes, and preagglutinated (modified) starch derived from wheat, corn, rice and potatoes, sodium starch glycolate; natural gums such as acacia gum, gelatin and tragacanth; derivatives of sea weed such as alginic acid, sodium alginate and ammonium calcium alginate, cellulose or derivatives thereof such as hydroxypropyl cellulose, L-hydroxypropyl cellulose, low-substituted hydroxypropyl cellulose, methyl cellulose, sodium carboxymethylcellulose, hydroxypropyl methylcellulose, and polyvinylpyrrolidone (crospovidone) in particular povidone K25. Preferably the binder is a polymer, more preferably a gel-forming polymer, still more preferably the binder is a cellulose or a derivative thereof, still more preferably L-hydroxypropyl cellulose, and most preferably a combination of L-hydroxypropyl cellulose and hydroxypropyl cellulose.

Hydroxypropyl cellulose is a partially substituted poly(hydroxypropyl) ether of cellulose. It may contain not more than 0.6% of silica or another suitable anticaking agent. Hydroxypropyl cellulose is commercially available in a number of different grades that have various solution viscosities. Molecular weight ranges from 50000-1250000. Hydroxypropylcellulose is partly O-(2-hydroxypropylated) cellulose. It contains 53.4% to 80.5% of hydroxypropoxy groups with reference to the dried substance. The average grade of polymerization ranges from 200 to 300. The molar grade of substitution is around 4.
"Low-substituted hydroxypropyl cellulose" (L-HPC or LHPC) is a low-substituted poly(hydroxypropyl) ether of cellulose. It is commercially available in a number of different grades that have different particle sizes and substitution levels.
Low-substituted hydroxypropyl cellulose contains 5% to 16% hydroxypropoxy groups with reference to the dried substance. The molar grade of substitution is < 1. In particular low-substituted hydroxypropyl cellulose is a low-substituted O-(2-hydroxypropylated) cellulose contains not less than 5.0% and not more than 16.0% of hydroxypropoxy groups (-OCH₂CHOHCH₃), calculated on the dried basis.
Low-Substituted Hydroxypropyl Cellulose is a low-substituted O-(2-hydroxypropylated) cellulose contains not less than 5.0% and not more than 16.0% of hydroxypropoxy groups (-OCH₂CHOHCH₃), calculatedon the dried basis.

"Povidone" is synonymously used for polyvinylpyrrolidone (PVP).
Polyvinylpyrrolidone consists of linear polymers of 1-ethenylpyrollidin-2-one. The different types of polyvinylpyrrolidone are characterized by the viscosity of their solutions, expressed by the K value. Polyvinylpyrrolidone is present as a white to yellowish white powder or flake and is readily soluble in water. The K value is a common classification in the plastics industry and is directly related to the average molar mass of the polymer. This makes it possible to deduce indirectly from the K value the degree of polymerization and thus the chain length. Povidone K25, povidone K30 or povidone K90 is commercially available. Preferably, povidone K25 is used as a binder. The approximate average molecular weight of povidone K25 is 30,000 g/mol (Da) between 28,000 g/mol (Da) to 34,000 g/mol (Da).

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a binder.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one binder.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a binder, and wherein the binder is polyvinylpyrrolidone.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one polyvinylpyrrolidone.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a binder, wherein the binder is polyvinylpyrrolidone, and wherein the polyvinylpyrrolidone is povidone K25.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and povidone K25.

Moreover, the systemic formulation according to the invention can comprise a binder and/or a polymeric precipitation inhibitor.
A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of binder and polymeric precipitation inhibitor.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one binder and/or at least one polymeric precipitation inhibitor.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a binder and polymeric precipitation inhibitor. Preferably, at least one excipient functions as a binder and a polymeric precipitation inhibitor at the same time. Thus, at least one excipient is a binder and polymeric precipitation inhibitor.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one compound being a binder and polymeric precipitation inhibitor.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one binder and at least one polymeric precipitation inhibitor.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of binder and polymeric precipitation inhibitor, and wherein the polymeric precipitation inhibitor is cellulose, a cellulose derivative, a combination of cellulose and a derivative or combination of cellulose derivatives.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one binder and/or at least one polymeric precipitation inhibitor, wherein the polymeric precipitation inhibitor is cellulose, a cellulose derivative, a combination of cellulose and a derivative or combination of cellulose derivatives.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of binder and polymeric precipitation inhibitor, and wherein the polymeric precipitation inhibitor is L-hydroxypropyl cellulose, hydroxypropyl cellulose, or a combination of L-hydroxypropyl cellulose and hydroxypropylcellulose.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one binder and/or at least one polymeric precipitation inhibitor, wherein the polymeric precipitation inhibitor is L-hydroxypropyl cellulose, hydroxypropyl cellulose, or a combination of L-hydroxypropyl cellulose and hydroxypropyl cellulose.

An embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of binder and polymeric precipitation inhibitor, wherein the polymeric precipitation inhibitor is L-hydroxypropyl cellulose, hydroxypropyl cellulose or a combination of L-hydroxypropyl cellulose, and wherein the binder is L-hydroxypropyl cellulose, hydroxypropyl cellulose, a combination of L-hydroxypropyl cellulose and hydroxypropyl cellulose, and/or polyvinylpyrrolidone.

An embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one polymeric precipitation inhibitor selected from the group comprising or consisting of L-hydroxypropyl cellulose and hydroxypropyl cellulose and at least one binder selected from the group L-hydroxypropyl cellulose, hydroxypropyl cellulose and hydroxypropyl cellulose, and/or polyvinylpyrrolidone.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a binder and/or at least one excipient is a polymeric precipitation inhibitor, and wherein the polymeric precipitation inhibitor is cellulose, a cellulose derivative, a combination of cellulose and a cellulose derivative or combination of cellulose derivatives, and the binder is cellulose, a cellulose derivative, a combination of cellulose and a derivative or combination of cellulose derivatives, and/or povidone K25.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one binder and/or at least one polymeric precipitation inhibitor, and wherein the polymeric precipitation inhibitor is cellulose, a cellulose derivative, a combination of cellulose and a derivative or combination of cellulose derivatives, and the binder is cellulose, a cellulose derivative, a combination of cellulose and a derivative or combination of cellulose derivatives, and/or povidone K25.

Another preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a binder and/or at least one excipient is a polymeric precipitation inhibitor, wherein the polymeric precipitation inhibitor is L-hydroxypropyl cellulose, hydroxypropyl cellulose or a combination of L-hydroxypropyl cellulose, and wherein the binder is L-hydroxypropyl cellulose, hydroxypropyl cellulose, a combination of L-hydroxypropyl cellulose and hydroxypropyl cellulose, and/or povidone K25.

Another preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one binder and/or at least one polymeric precipitation inhibitor, wherein the polymeric precipitation inhibitor is L-hydroxypropyl cellulose, hydroxypropyl cellulose or a combination of L-hydroxypropyl cellulose, and wherein the binder is L-hydroxypropyl cellulose, hydroxypropyl cellulose, a combination of L-hydroxypropyl cellulose and hydroxypropyl cellulose, and/or povidone K25.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a binder and/or at least one excipient is a polymeric precipitation inhibitor, wherein the polymeric precipitation inhibitor is L-hydroxypropyl cellulose, hydroxypropyl cellulose, or a combination of L-hydroxypropyl cellulose and hydroxypropyl cellulose, wherein the binder is povidone K25.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one binder and/or at least one polymeric precipitation inhibitor, wherein the polymeric precipitation inhibitor is L-hydroxypropyl cellulose, hydroxypropyl cellulose, or a combination of L-hydroxypropyl cellulose and hydroxypropyl cellulose, wherein the binder is povidone K25.

The positive data of the pharmacokinetic study in human confirms the **high** bioavailability of compound (**I**). A high bioavailability in this context has to be understood in comparison to the animal studies which have been performed previously.

The STAM- and UUO mouse model studies demonstrate an anti-fibrotic effect on the liver and kidney being achieved by the administration of a systemic formulation containing the compound according to formula (**I**). Therefore, the drug of formula (I) administered systemically shows a high anti-fibrotic effect at the target site, and thereby indicates a systemic bioavailability. Thus, the administration of an oral formulation containing the drug of formula (I) exhibits a high anti-fibrotic effect at the target site, and thus a systemic availability (bioavailability) can be derived, i.e. the drug is absorbed at the target tissue.

The addition of an acidifier ensures the complete dissolution of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate in the stomach but the pH in the small intestine increases from 2 to 6, and thus the drug can precipitate before it can be absorbed by the small intestine. In order to ensure a complete dissolution of said compound in the small intestine, the formulation according to the invention contains preferably an acidifier, and/or a polymeric precipitation inhibitor. Moreover, it could be shown in example 9 that merely a dosage of 20 mg in human is sufficient to achieve drug concentrations in plasma, and thus a systemic availability.

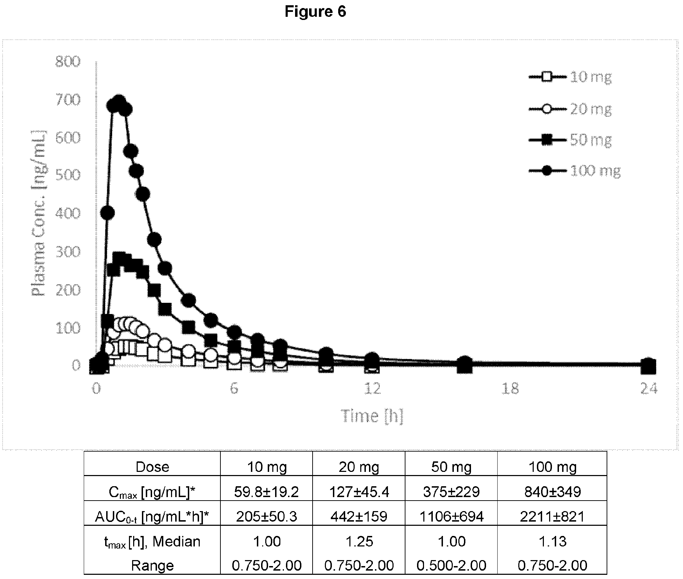

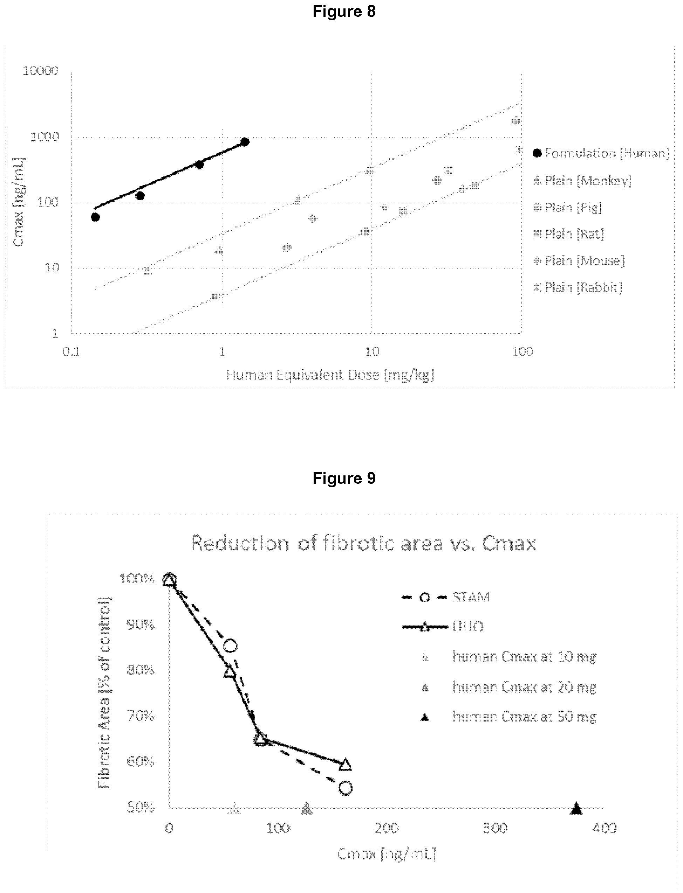

The pharmacokinetic studies show that (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate is absorbed in the small intestine and a low dose of 20-50 mg is needed to achieve a therapeutic effective drug concentration in human (example 7, Figure 6). In addition, an anti-fibrotic effect can already be achieved with a human dosage of 20 mg (example 9 and Figure 9).

Moreover, off-target effects could not be observed. As aforementioned, this is particularly surprising due to the fact that TG2 is ubiquitously expressed in almost all cell types and cell compartments, it is present on the cell surface and gets secreted to the extracellular matrix, and is present in various organs, and thus it could be envisioned that off-target effects would be most likely.

Thus, the systemic formulation according to the invention can comprise an acidifier and/or a polymeric precipitation inhibitor. Preferably, the systemic formulation comprises an acidifier and a polymeric precipitation inhibitor.

A preferred embodiment according to the invention is directed to a systemic formulation containing or consisting of a solvate, hydrate or a pharmaceutically acceptable salt thereof, and at least one excipient, wherein at least one excipient is selected from the group comprising or consisting of acidifier, and polymeric precipitation inhibitor.

A preferred embodiment of the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier and at least one polymeric precipitation inhibitor.

A preferred embodiment of the invention is thus related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, adipic acid and at least one polymeric precipitation inhibitor.

A preferred embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, anda polymeric precipitation inhibitor, and wherein the acidifier is adipic acid, and the polymeric precipitation inhibitor is selected from the group comprising or consisting of cellulose and cellulose derivative.

A preferred embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one acidifier and at least one polymeric precipitation inhibitor, wherein the acidifier is adipic acid, and the polymeric precipitation inhibitor is selected from the group comprising or consisting of cellulose and cellulose derivative.

A preferred embodiment of the invention is thus related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier and at least one polymeric precipitation inhibitor selected from the group comprising or consisting of L-hydroxypropyl cellulose and hydroxypropyl cellulose.

A preferred embodiment of the invention is thus related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid and at least one polymeric precipitation inhibitor selected from the group comprising or consisting of L-hydroxypropyl cellulose and hydroxypropyl cellulose.

A preferred embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, and polymeric precipitation inhibitor, and wherein the acidifier is adipic acid, and the polymeric precipitation inhibitor is L-hydroxypropyl cellulose, hydroxypropyl cellulose or a combination of L-hydroxypropyl cellulose and hydroxypropyl cellulose.

A preferred embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one acidifier, and/or at least one polymeric precipitation inhibitor, and wherein the acidifier is adipic acid, and the polymeric precipitation inhibitor is L-hydroxypropyl cellulose, hydroxypropyl cellulose or a combination of L-hydroxypropyl cellulose and hydroxypropyl cellulose.

A systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, wherein the systemic formulation further comprises at least one excipient, wherein at least one excipient is selected from the group comprising or consisting of acidifier and polymeric precipitation inhibitor, wherein the acidifier is selected from the group consisting of ascorbic acid, organic di-carboxylic acid such as oxalic acid, malonic acid, succinic acid, glutaric acid, tartaric acid, fumaric acid, maleic acid, malic acid, adipic acid, or glutamic acid, and organic tri-carboxylic acid such as citric acid, or sodium hydrogen citrate.

A systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, wherein the systemic formulation further comprises at least one excipient, wherein at least one excipient is selected from the group comprising or consisting of acidifier and polymeric precipitation inhibitor, wherein the acidifier is selected from the group consisting of ascorbic acid, organic di-carboxylic acid such as oxalic acid, malonic acid, succinic acid, glutaric acid, tartaric acid, fumaric acid, maleic acid, malic acid, adipic acid, or glutamic acid, and organic tri-carboxylic acid such as citric acid, or sodium hydrogen citrate, and wherein the polymeric precipitation inhibitor is selected from the group consisting of sugar, such as sucrose; polysaccharides such as xanthan gum, guar gum, carrageenan, starches derived from wheat, corn, rice and potatoes, and preagglutinated starch derived from wheat, corn, rice and potatoes, sodium starch glycolate; polyacrylic acids; natural gums such as acacia gum, gelatin and tragacanth; derivatives of sea weed such as alginic acid, sodium alginate and ammonium calcium alginate, cellulose or derivative thereof such as hydroxypropyl cellulose, L-hydroxypropyl cellulose, methyl cellulose and sodium carboxymethylcellulose and hydroxypropyl methylcellulose, or polyvinylpyrrolidone.

The systemic formulation according to the invention can comprise an acidifier, a polymeric precipitation inhibitor, and/or a binder.
An embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, polymeric precipitation inhibitor and binder.

A preferred embodiment according to the invention is directed to a systemic formulation containing or consisting of (S,E)-methyl-7-(1-(2-(2-ethylbutylamino)-2-oxoethyl)-2-oxo-1 ,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier, at least one acidifier, at least one polymeric precipitation inhibitor and at least one binder.

A more preferred embodiment according to the invention is directed to a systemic formulation containing or consisting of (S,E)-methyl-7-(1-(2-(2-ethylbutylamino)-2-oxoethyl)-2-oxo-1 ,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier, at least one acidifier, at least one polymeric precipitation inhibitor and at least one binder, wherein the acidifier is adipic acid, the polymeric precipitation inhibitor is L-hydroxypropyl cellulose, hydroxypropyl cellulose or a combination of L-hydroxypropyl cellulose and hydroxypropyl cellulose, and wherein the binder is polyvinylpyrrolidone.

The systemic formulation according to the invention can comprise a disintegrant. Thus, the excipient can be a disintegrant. The term **"disintegrant"** refers to materials added to the composition in order to support disintegration of the formulation and release of the active pharmaceutical ingredient. Suitable disintegrants include starches, modified starches which are soluble in cold water, such as sodium carboxymethyl starch; cellulose derivatives such as methylcellulose and sodium carboxymethylcellulose, microcrystalline cellulose and crosslinked microcrystalline cellulose such as sodium croscarmellose; alginates such as alginic acid and sodium alginate; clays such as bentonites and foaming mixtures; effervescent compounds such as combinations of citric acid, tartaric acid, sodium citrate, disodium hydrogen citrate, monosodium citrate, sodium and/or potassium hydrogen carbonate that react in the presence of water to give carbon dioxide. Preferably, the disintegrant is sodium croscarmellose. Microcrystalline cellulose is a purified, partially depolymerized cellulose that occurs as a white, odorless, tasteless, crystalline powder composed of porous particles. It is manufactured by treating alpha-cellulose, obtained as a pulp from fibrous plant material, with mineral acids. Several different grades are commercially available that differ in their method of manufacture, particle size, moisture, flow, and other physical properties. The larger-particle-size-grades generally provide better flow properties. Low-moisture-grades are used with moisture-sensitive materials. Higher-density grades have improved flowabilities.
The microcrystalline cellulose used herein can have a nominal mean particle size of 100 µm and a moisture content of ≤ 5.0%.

An embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a disintegrant.

An embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one disintegrant.

The systemic formulation according to the invention can comprise an acidifier, a polymeric precipitation inhibitor, a binder and/or a disintegrant.
An embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, polymeric precipitation inhibitor, binder and disintegrant.

The systemic formulation according to the invention can comprise a lubricant/glidant. The excipient can thus be a lubricant/glidant. Lubricants/glidants are materials preventing caking, improving the flow characteristics of granulates so that the flow is smooth and uniform, and reducing t the friction between surfaces in direct contact in order to allow for the tablet, granulate, etc. to be released from the casting mold or pressing mold, after compression. Lubricants/glidants include sodium benzoate, metallic stearate such as magnesium stearate, calcium stearate, or potassium stearate, stearic acid, high melting point waxes, inorganic lubricants/glidants such as silicon dioxide and talc and other lubricants/glidants such as sodium oleate, and polyethylene glycols. Preferably, the lubricant/glidant is talc or silicon dixoide. Due to the fact that lubricants/glidants have to be present on the surface of the granules as well as between the granules and parts of the equipment they are typically added during the last step prior to encapsulation or compression.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a lubricant/glidant.

A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one lubricant/glidant.

The systemic formulation according to the invention can comprise or consist of an acidifier, a polymeric precipitation inhibitor, a binder and/or a lubricant/glidant.
A preferred embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, polymeric precipitation inhibitor, binder, and lubricant/glidant.

A preferred embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, polymeric precipitation inhibitor, binder, and lubricant/glidant.

The systemic formulation according to the invention can comprise or consist of an acidifier, polymeric precipitation inhibitor, a binder, a disintegrant, and/or a lubricant/glidant.

A preferred embodiment according to the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, polymeric precipitation inhibitor, binder, disintegrant, and lubricant/glidant.

Furthermore, the systemic formulation according to the invention can also comprise as an excipient diluents/fillers/binders, sweetening agents, flavoring agents, buffering agents, antioxidants, emulsifiers, solubilizer/wetting agent and/or preservatives.

A suitable diluent/filler/binder is a substance which usually forms the largest part of the composition or dosage form. A suitable diluent/filler/binder includes sugars such as lactose, sucrose, mannitol and sorbitol; starches derived from wheat, corn, rice and potatoes; and cellulose such as microcrystalline cellulose, calcium hydrogen phosphate dihydrate, and calcium sulfate. Preferably, the diluent/filler/binder is cellulose and/or mannitol. Most preferably, the diluent/filler/binder is microcrystalline cellulose and/or mannitol. Preferably, the diluent/filler/binder is microcrystalline cellulose when the formulation is a tablet, and the diluent/filler/binder is mannitol when the formulation is a capsule.

The addition of mannitol further increases the porosity and therefore wettability of the granules. A preferred embodiment of the invention is therefore directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is a diluent/filler/binder.

The systemic formulation according to the invention can comprise or consists of an acidifier, a polymeric precipitation inhibitor, a binder, a disintegrant, a lubricant/glidant and/or a diluent/filler/binder.
An embodiment of the invention is directed to a formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, polymeric precipitation inhibitor, binder, disintegrant, lubricant/glidant and diluent/filler/binder.

An embodiment of the invention is directed to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier, at least one polymeric precipitation inhibitor, at least one a binder, at least one disintegrant, at least one lubricant/glidant and at least one diluent/filler/binder.

The preferred preparations are provided in an administrable form suitable for oral application, such as tablets such as uncoated tablets, coated tablets, effervescent tablets, soluble tablets, chewable tablets, oral lyophilisates, lozenges, pastilles, compressed lozenges, sublingual tablets, buccal tablets, granules, effervescent granules and capsules. More preferably, the oral formulation is a tablet or capsule. Uncoated and coated, and capsules, either hard or soft are the most preferred pharmaceutical formulations.

An embodiment of the present invention is directed to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, wherein the systemic formulation is a tablet, capsule, powder, or granule.

The systemic formulation according to the invention can comprise other ingredients such a unavoidable impurities, ingredients for the capsule including colorants of the capsule. Also in a tablet, a colorant as other ingredient can be present.
Furthermore, components used for coating a tablet are also encompassed by the term "other ingredients".

The shell of the capsule can comprise a colorant. As used herein, the term "colorant" includes pigments such as white pigments. The colorant can be among others iron oxide in particular iron(III)oxide, iron(II,III) oxide or hydrated ferric oxide or titanium dioxide.

"Tablet" means a compressed solid dosage form containing at least one active pharmaceutical ingredient with suitable excipients. The tablet can be produced by compressing mixtures or granulates obtained by wet granulation, dry granulation or compaction, which are known to the one skilled in the art.

The term "capsule" refers to a special container or shell composed of methylcellulose, polyvinyl alcohols or gelatins or denatured gelatins or starches, in which the active agents can be enclosed. Typically, hard shell capsules are prepared from hydroxypropyl methylcellulose or from mixtures of porcine bone and skin gelatins having comparatively high gel strength. The shell of the capsule can contain small amounts of colorants, opacifiers, softening agents and preservatives. "Soft shell capsules" contains gelatin as a basic polymer, one or more softening agents such as glycerol or sorbitol in a higher amount as well as water. In general, the amount of the softening agent is 20 - 30% by weight of the capsule shell, the amount of the gelatin is 40 - 45% by weight of the capsule shell, and amount of water is 30 - 35% by weight of the capsule shell. After the drying of the capsule, the amount of water is 7 - 8% by weight of the capsule shell.
The capsule shell can comprise gelatine, hydroxypropyl methylcellulose (HMPC), polysaccharides such as starch, and carrageenan; and/or synthetic polymers such as compolymers of polyvinylalcohol. Furthermore, the shell of the capsule can comprise a colorant. As used herein, the term "colorant" includes pigments such as white pigments. The colorant can be among others iron oxide in particular iron(III)oxide, iron(II,III) oxide or hydrated ferric oxide, titanium dioxide, natural dyes, azo and xanthane compounds. Moreover, the capsule shell may comprise a preservative such as p-hydroxybenzoic acid esters or means to improve the flavour such as ethylvanillin. In addition, the capsule shell can comprise a surfactant such a sodium lauryl sulfate.

"Powders" for compositions refer to powder mixtures/blends containing the active components and suitable excipients which can be suspended in water or juices prior to use. Spherical-shaped granules are also referred to pellets or beads.

"Granules" refer to dry and solid grains. Each grain represents an agglomerate of powder particles.

While the wrapping or embedding method drug particles are treated, the coating method is related to the dosage form itself. Tablets, the center of dragees or capsules are coated with coating layer, wherein excipients such as derivative of cellulose, cellulose ether such as hydroxypropyl methylcellulose (HMPC), synthetic polymers, shellac, corn protein zein or other polysaccharides and anionic copolymers of methacrylic acid and methyl methacrylate. The coating can further comprise colorants such as titanium dioxide, iron(III)oxide, iron(II,III) oxide or hydrated ferric oxide, lactose monohydrate, and or carnauba wax. Also capsule can be coated.

Sustained-release-type formulation are known in the state of the art for the provision of a controlled release rate of any one or more components or active components, in order to optimize the therapeutic effect, i.e. the inhibitory activity and the like. The pharmacological optimal concentration is guaranteed for a certain time above the period of effect of a single dosage. Suitable dosage forms for sustained release include layered tablets containing layers with varying degradation rates or controlled release polymeric matrices impregnated with the active components and in the form of a tablet or capsule containing such impregnated or encapsulated porous polymeric matrices. A sustained-release type formulation would impede a fast-release of the compound. Herein, it is desired that a high concentration of the drug is quickly released to the target site after the administration. Consequently, sustained-release type formulations are not preferred and should actually be avoided for the purposes of the present invention, because preliminary results indicate that such formulations cannot provide the required high drug concentration according to the present invention.

The systemic formulation according to the invention can be in form of a capsule or tablet, i.e. active agent and the excipient can be filled in the capsule. An embodiment according to the invention is a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, wherein the systemic formulation is in form of a capsule or tablet.

A preferred embodiment according to the invention is a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, and polymeric precipitation inhibitor, wherein the systemic formulation is in form of a capsule or tablet.

A preferred embodiment according to the invention is a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier, and at least one polymeric precipitation inhibitor, wherein the systemic formulation is in form of a capsule or tablet.

Another preferred embodiment according to the invention is a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least one excipient, wherein at least one excipient is selected from the group comprising or consisting of acidifier, polymeric precipitation inhibitor, and binder, wherein the systemic formulation is in form of a capsule or tablet.

Another preferred embodiment according to the invention is a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier, at least one polymeric precipitation inhibitor, and at least one binder, wherein the systemic formulation is in form of a capsule or tablet.

Another preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least at excipient, wherein at least one excipient is selected from the group comprising or consisting of acidifier, polymeric precipitation inhibitor, binder, disintegrant, and a lubricant/glidant, wherein the systemic formulation is in form of a capsule or tablet.

Another preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier, at least one polymeric precipitation inhibitor, at least one binder, at least one disintegrant, and at least one lubricant/glidant, wherein the systemic formulation is in form of a capsule or tablet.

Another preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and at least at excipient, wherein at least one excipient is selected from the group comprising or consisting of acidifier, polymeric precipitation inhibitor, diluent/filler/binder, disintegrant, lubricant/glidant, and diluent/filler/binder, wherein the systemic formulation is in form of a capsule or tablet.

Another preferred embodiment according to the invention is related to a systemic formulation containing or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, at least one acidifier, at least one polymeric precipitation inhibitor, at least one binder, at least one disintegrant, at least one lubricant/glidant, and at least one diluent/filler/binder, wherein the systemic formulation is in form of a capsule or tablet.

The preferred pharmaceutical formulation is for oral administration. Therefore, preferred pharmaceutical formulations are systemic formulation in form of an enteral or parenteral formulation for oral administration. Consequently, especially capsules and tablets are the most preferred enteral or parenteral formulation for oral administration and especially these capsules and tablets which ensure fast release of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate, and thereby ensuring high drug concentrations. Therefore, sustained release formulations are not suitable and should actually not be used for the purposes of the present invention.

Moreover, pharmaceutical formulations for oral administration containing adipic acid are preferred. More preferred are systemic formulations in form of an enteral or parenteral formulation for oral administration containing adipic acid. Most preferred are capsules and tablets for oral administration containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and adipic acid.

Furthermore, in order to further improve the performance of the formulation the specific PSD (particle size distribution), and/or PSR (particle size range) can be adapted.

Therefore, an embodiment according to the invention is directed to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate of the formula (I): 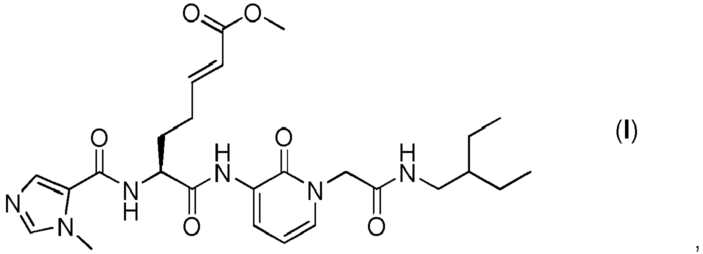 or a solvate, hydrate or a pharmaceutically acceptable salt of formula (I), wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size range from 0.1 to 100 µm.

It is furthermore preferred that the particle size of the (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof is within the range of 0.1 µm to 100 µm, preferably in the range of 0.5 µm to 50 µm and more preferably in the range of 1.0 µm to 20 µm. Thus, the particle size range (PSR) of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof is from 0.1 µm to 100 µm, from 0.5 µm to 50 µm, or from 1.0 µm to 20 µm. Preferably, the particle size of the drug according to formula (I) is ≤10 µm.

Another preferred embodiment according to the invention is a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, and L-hydroxypropyl cellulose, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size range from 0.1 µm to 100 µm.

Therefore, an embodiment according to the invention is directed to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate of the formula (I): 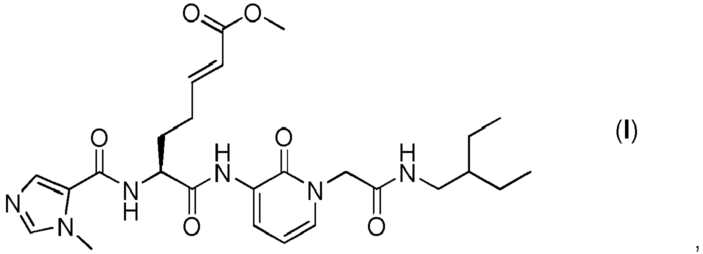 or an enantiomer, a solvate, hydrate or a pharmaceutically acceptable salt of formula (I), wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size distribution which is defined by d(0.95) ≤ 25 µm, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof is preferably micronized.

Moreover it is preferred that the particle size distribution of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof is characterized by d(0.1) from 0.1 to 5 µm, d(0.5) from 0.3 to 10 µm, d(0.95) from 3 to 25 µm, more preferably d(0.1) from 0.2 to 3 µm, d(0.5) from 0.4 to 7.5 µm and d(0.95) from 2 to 15 µm, and most preferably d(0.1) from 0.3 to 3 µm, d(0.5) from 0.5 to 5 µm and d(0.95) from 1 to 10 µm
The particle size distribution is measured by laser light diffraction (Malvern analysis, sample dispersed in n-hexane and sorbitane monooleate). Thereby, the laser light is scattered in dependence of the particle size. A diffraction pattern results from the angle dependent scattered light intensity, the particle size can be calculated.

The parameter d(0.1) refers to the diameter at which 10% of the total volume of particles in the sample is comprised of particles with a diameter less than the indicated value or range of values when analysed by laser diffraction (Malvern analysis, sample dispersed in n-hexane and sorbitane monooleate). Thus d(0.1) = 0.1 to 5 µm means that the upper limit of the particle size range defining the 10% of smallest particles in the sample is between 0.1 µm to 5 µm. Thus 10% of the total particles have a particle size of not more than d(0.1) meaning in this case that they have a maximum size of 0.1 µm to 5 µm.

Accordingly the parameter d(0.5) refers to the diameter at which 50% of the total volume of particles in the sample is comprised of particles with a diameter less than the indicated value or range of values when analysed by laser diffraction (Malvern analysis, sample dispersed in n-hexane and sorbitane monooleate). Thus d(0.5) = 0.3 to 10 µm means that the upper limit of the particle size range defining the 50% of smallest particles in the sample is between 0.3 µm to 10 µm. Thus 50% of the total particles have a particle size of not more than d(0.5) meaning in this case that they have a maximum size of 0.3 µm to 10 µm.

Accordingly the parameter d(0.95) refers to the diameter at which 95% of the total volume of particles in the sample is comprised of particles with a diameter less than the indicated value or range of values when analysed by laser diffraction (Malvern analysis, sample dispersed in n-hexane and sorbitane monooleate). Thus d(0.95) = 3 to 25 µm means that the upper limit of the particle size range defining the 95% of smallest particles in the sample is between 3 µm to 25 µm. Thus 95% of the total particles have a particle size of not more than d(0.95) meaning in this case that they have a maximum size of 3 µm to 25 µm.

Another embodiment of the present invention is directed to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, and L-hydroxypropyl cellulose, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size distribution which is defined by d(0.95) ≤ 25 µm.

A preferred embodiment of the present invention is directed to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size distribution which is defined by d(0.1) from 0.1 to 5 µm, d(0.5) from 0.3 to 10 µm, and d(0.95) from 3 to 25 µm.

A more preferred embodiment according to the invention is a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, and L-hydroxypropyl cellulose, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size distribution which is defined by d(0.1) from 0.1 to 5 µm, d(0.5) from 0.3 to 10 µm, and d(0.95) from 3 to 25 µm.

A still more preferred embodiment according to the invention is a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, and L-hydroxypropyl cellulose, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size distribution which is defined by d(0.1) from 0.2 to 3 µm, d(0.5) from 0.4 to 7.5 µm and d(0.95) from 2 to 15 µm.

A even more preferred embodiment according to the invention is a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, L-hydroxypropyl cellulose, sodium croscarmellose, and talc, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size distribution which is defined by d(0.1) from 0.1 to 5 µm, d(0.5) from 0.3 to 10 µm, and d(0.95) from 3 to 25 µm.

A particularly preferred embodiment according to the invention is a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, L-hydroxypropyl cellulose, sodium croscarmellose, talc, gelatine and titanium dioxide, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate have a particle size distribution which is defined by d(0.1) from 0.1 to 5 µm, d(0.5) from 0.3 to 10 µm, and d(0.95) from 3 to 25 µm.

Another particularly preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, L-hydroxypropyl cellulose, povidone K25, sodium croscarmellose, microcrystalline cellulose and silicon dioxide, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate have a particle size distribution which is defined by d(0.1) from 0.1 to 5 µm, d(0.5) from 0.3 to 10 µm, and d(0.95) from 3 to 25 µm.

Therefore, an embodiment according to the invention is directed to a systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate of the formula (I): 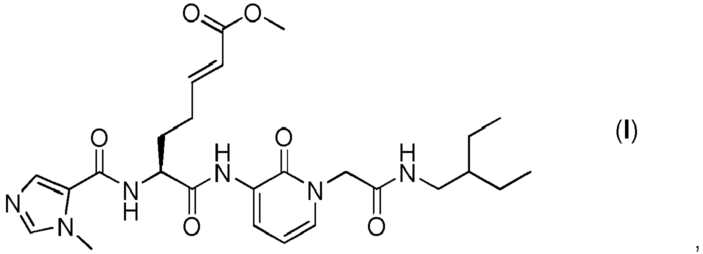 or an enantiomer, a solvate, hydrate or a pharmaceutically acceptable salt of formula (I), wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size range from 0.1 to 100 µm, and a particle size distribution which is defined by d(0.95) ≤ 25 µm.

Thus, preferred are systemic formulations containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxo-hept-2-enoate or an enantiomer, a solvate, hydrate or a pharmaceutically acceptable salt thereof, wherein the (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxo-hept-2-enoate or enantiomer, solvate, hydrate or pharmaceutically acceptable salt thereof is in form of particles having a particle size distribution which is defined by d(0.95) ≤ 25 µm.

A preferred embodiment according to the invention is a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, and L-hydroxypropyl cellulose, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size range from 0.1 to 100 µm, and a particle size distribution which is defined by d(0.95) ≤ 25 µm.

A more preferred embodiment according to the invention is a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate, or an enantiomer, a solvate, hydrate or a pharmaceutically acceptable salt thereof adipic acid, L-hydroxypropyl cellulose, hydroxypropyl cellulose, mannitol, sodium croscarmellose, and talc, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size range from 0.1 to 100 µm, and a particle size distribution which is defined by d(0.95) ≤ 25 µm.
A particularly preferred embodiment according to the invention is a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, L-hydroxypropyl cellulose, hydroxypropyl cellulose, mannitol, sodium croscarmellose, talc, gelatine and titanium dioxide, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size range from 0.1 to 100 µm, and a particle size distribution which is defined by d(0.95) ≤ 25 µm.

Another particularly preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, adipic acid, L-hydroxypropyl cellulose, povidone K25, sodium croscarmellose, microcrystalline cellulose and silicon dioxide, wherein (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate particles have a particle size range from 0.1 to 100 µm, and a particle size distribution which is defined by d(0.95) ≤ 25 µm.

The systemic formulation can contain (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or enantiomer, solvate, hydrate, or a pharmaceutically acceptable salt in an amount of at least 0.01 mg, preferably at least 0.1 mg, more preferably at least 0.5 mg, even more preferably at least 1 mg, even more preferably at least 2 mg, even more preferably at least 3 mg, even more preferably at least 4 mg, even more preferably at least 5 mg, even more preferably 0.01 mg to 1000 mg, even more preferably 0.05 mg to 900 mg, even more preferably 0.10 mg to 800 mg, still more preferably 0.2 mg to 700 mg, still more preferably 0.3 mg to 600 mg, still more preferably 0.4 mg to 500 mg, still more preferably 0.5 mg to 500 mg, still more preferably 0.6 mg to 450 mg, still more preferably 0.7 mg to 400 mg, still more preferably 0.8 mg to 375 mg, still more preferably 0.9 mg to 350 mg, still more preferably 1.0 mg to 300 mg, still more preferably 1.25 mg to 300 mg, still more preferably 1.5 mg to 275 mg, still more preferably 1.75 mg to 250 mg, still more preferably 2.0 mg to 225 mg, mg, still more preferably 2.25 to 220 mg, still more preferably 2.5 to 220 mg, still more preferably 2.75 mg to 215 mg, still more preferably 3.0 mg to 210 mg, still more preferably 3.75 mg to 205 mg, still more preferably 4.0 mg to 205 mg, 4.5 mg to 200 mg, most preferably 5 mg to 200 mg per formulation.

The systemic formulation can contain (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or enantiomer, solvate, hydrate or a pharmaceutically acceptable salt, solvate or hydrate in an amount of 0.1 wt% to 99 wt%, preferably 0.2 wt% to 90 wt%, more preferably 0.3 wt% to 85 wt%, even more preferably 0.4 wt% to 80 wt%, even more preferably 0.5 wt% to 75 wt%, even more preferably 0.6 wt% to 70 wt%, even more preferably 0.7 wt% to 65 wt%, even more preferably 0.8 wt% to 60 wt%, even more preferably 0.9 wt% to 55 wt%, even more preferably 1 wt% to 50 wt%, even more preferably 1 wt% to 45 wt%, even more preferably 1.25 wt% to 45 wt%, even more preferably 1.5 wt% to 40 wt%, even more preferably 1.75 wt% to 35 wt%, even more preferably 2 wt% to 34 wt%, even more preferably 2.25 wt% to 33 wt%, even more preferably 2.5 wt% to 32 wt%, and most preferably 2.5 wt% to 31 wt%, even more preferably 2.5 wt% to30.5 wt%, and even more preferably 2.6 wt% to 30.3 wt%, even more preferably 3 wt% to 30 wt%, even more preferably 3.5 wt% to 29 wt%, even more preferably 4 wt% to 28 wt%, even more preferably 4 wt% to 27 wt%, even more preferably 4.5 wt% to 27 wt%, and most preferably 5 wt% to 27 wt%. "Wt%" (weight percent") refers to the weight percent in the composition.

The amount of the acidifier can range from 0.1 wt% to 80 wt%, preferably from 0.5 wt% to 77.5 wt%, more preferably from 1 wt% to 75 wt%, more preferably from 1.5 wt% to 72.5 wt%, more preferably from 2 wt% to 70 wt%, more preferably from 2.5 wt% to 62.5 wt%, more preferably from 3 wt% to 57.5 wt%, more preferably from 3.5 wt% to 55 wt%, even more preferably from 4 wt% to 55 wt%, even more preferably from 4.5 wt% to 55 wt%, even more preferably from 5 wt% to 54 wt%, even more preferably from 5.5 wt% to 53 wt%, even more preferably from 6 wt% to 52 wt%, even more preferably from 6.5 wt% to 51 wt%, even more preferably from 7 wt% to 50 wt%, even more preferably from 8 wt% to 49 wt%, even more preferably from 8.5 wt% to 49 wt%, and most preferably from 9 wt% to 49 wt%.

Moreover, the amount of the acidifier can range from 1.00 mg to 500 mg, more preferably from 1.25 mg 495 mg, still more preferably from 1.50 mg to 490 mg, still more preferably from 1.75 mg to 485 mg, still more preferably from 2.00 mg to 480 mg, still more preferably from 2.25 mg to 475 mg, still more preferably from 2.50 mg to 470 mg, still more preferably from 3.0 mg to 465 mg, still more preferably from 3.25 mg to 460 mg, still more preferably from 3.5 mg to 455 mg, even more preferably from 3.75 mg to 450 mg, even more preferably from 4.00 mg to 445 mg, even more preferably from 4.25 mg to 440 mg, still more preferably from 4.5 mg to 435 mg, still more preferably from 4.75 mg to 430 mg, still more preferably from 5.0 mg to 425 mg, still more preferably from 5.25 mg to 420 mg, still more preferably from 5.5 mg to 415 mg, still more preferably from 5.75 mg to 410 mg, still more preferably from 6.0 mg to 410 mg, still more preferably from 6.25 mg to 405 mg, still more preferably from 6.5 mg to 400 mg, still more preferably from 6.75 mg to 395 mg, still more preferably from 7.0 mg to 390 mg, still more preferably from 7.5 mg to 390 mg, still more preferably from 7.75 mg to 385 mg, still more preferably from 8.0 mg to 380 mg, still more preferably from 8.5 mg to 375 mg, still more preferably from 9 mg to 370 mg, still more preferably from 9 mg to 365 mg, still more preferably from 9 mg to 360 mg, still more preferably from 9 mg to 350 mg, still more preferably from 9 mg to 325 mg, still more preferably from 9 mg to 300 mg, still more preferably from 9 mg to 250 mg, still more preferably from 9 mg to 200 mg, and most preferably from 9 mg to 180 mg.
Furthermore, a mass ratio of the acidifier relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof can range from 15 to 0.1 m/m, preferably from 14.5 to 0.2 m/m, more preferably from 14.0 to 0.3 m/m, still more preferably from 13.5 to 0.4 m/m, still more preferably from 13.0 to 0.5 m/m, still more preferably from 12.5 to 0.6 m/m, still more preferably from 12.0 to 0.7 m/m, still more preferably from 11.75 to 0.8 m/m, still more preferably from 11.5 to 0.9 m/m, still more preferably from 11.5 to 1.0 m/m, still more preferably from 11.5 to 1.1 m/m, still more preferably from 11.5 to 1.2 m/m, still more preferably from 11.5 to 1.3 m/m, still more preferably from 11.5 to 1.4 m/m, still more preferably from 11.5 to 1.5 m/m, still more preferably from 11.5 to 1.6 m/m, still more preferably from 11.5 to 1.7 m/m, and most preferably preferably from 11.5 to 1.8 m/m.

The amount of the polymeric precipitation inhibitor can vary from 0.1 wt% to 40 wt%, preferably 0.5 wt% to 39 wt%, more preferably 1 wt% to 38 wt%, still more preferably 1.25 wt% to 38 wt%, still more preferably 1.5 wt% to 37 wt%, still more preferably 1.75 wt% to 36 wt%, still more preferably 2 wt% to 35 wt%, still more preferably 1.5 wt% to 34 wt%, still more preferably 1.6 wt% to 33 wt%, still more preferably 1.7 wt% to 32 wt%, still more preferably 1.8 wt% to 31 wt%, still more preferably 3.5 wt% to 30 wt%, still more preferably 4 wt% to 29 wt%, still more preferably 4.5 wt% to 28.5 wt%, most preferably 5 wt% to 28.5 wt%.
Furthermore, the amount of the polymeric precipitation inhibitor can range from 1 mg to 100 mg, preferably from 1.5 mg to 95 mg, more preferably from 2 mg to 92.5 mg, still more preferably 2.5 mg to 90 mg, still more preferably 3 mg to 87.5 mg, still more preferably 3.5 mg to 85 mg, still more preferably 4 mg to 82.5 mg, still more preferably 4.5 mg to 80 mg, still more preferably 5 mg to 77.5 mg, still more preferably 5.5 mg to 75 mg, 6 mg to 72.5 mg, still more preferably 6.5 mg to 70 mg, still more preferably 7 mg to 65 mg, still more preferably 7.5 mg to 62.5 mg, still more preferably 8 mg to 60 mg, even more preferably from 8.5 mg to 57.5 mg, even more preferably from 9 mg to 55 mg, even more preferably from 9.5 mg to 52.5 mg, even more preferably from 9.75 mg to 52.5 mg, and most preferably from 10 mg to 50 mg.

Furthermore, a mass ratio of the polymeric precipitation inhibitor relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof can range from 0.05 to 10 m/m, preferably from 0.06 to 9.5 m/m, more preferably from 0.07 to 9.00 m/m, still more preferably from 0.08 to 8.50 m/m, still more preferably from 0.09 to 8.00 m/m, still more preferably from 0.1 to 7.5 m/m, still more preferably from 0.11 to 7.25 m/m, still more preferably from 0.12 to 7 m/m, still more preferably from 0.13 to 6.75 m/m, still more preferably from 0.14 to 6.5 m/m, still more preferably from 0.15 to 6.25 m/m, even more preferably from 0.16 to 6 m/m, even more preferably from 0.17 to 5.75 m/m, even more preferably from 0.18 to 5.5 m/m, even more preferably from 0.19 to 5.25 m/m, and most preferably from 0.20 to 5 m/m.

The amount of the binder can vary from 0 wt% to 40 wt%, preferably from 0 wt% to 35 wt%, more preferably from 0 wt% to 30 wt%, still more preferably from 0 wt% to 25 wt%, still more preferably from 0 wt% to 20 wt%, still more preferably from 0 wt% to 15 wt%, still more preferably from 0 wt% to 12 wt%, and most preferably from 0 wt% to 8.5 wt%.
Furthermore, the amount of the binder can range from 1.00 mg to 100 mg, preferably from 1.50 mg to 95 mg, more preferably from 2.00 mg to 92.5 mg, still more preferably 2.50 mg to 90 mg, still more preferably 3.00 mg to 87.5 mg, still more preferably 3.50 mg to 85 mg, still more preferably 4.00 mg to 82.5 mg, still more preferably 4.50 mg to 80 mg, still more preferably 5.00 mg to 77.5 mg, still more preferably 5.50 mg to 75 mg, 6.00 mg to 72.5 mg, still more preferably 6.50 mg to 70 mg, still more preferably 7.00 mg to 65 mg, still more preferably 7.50 mg to 62.5 mg, still more preferably 8.00 mg to 60 mg, even more preferably from 8.50 mg to 57.5 mg, even more preferably from 9.00 mg to 55.0 mg, even more preferably from 9.50 mg to 52.5 mg, even more preferably from 9.75 mg to 52.5 mg, and most preferably from 10 mg to 50 mg.

Furthermore, a mass ratio of the binder relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof can range from 0 to 10 m/m, preferably from 0.05 to 9.5 m/m, more preferably from 0.06 to 9.00 m/m, still more preferably from 0.07 to 8.50 m/m, still more preferably from 0.08 to 8.00 m/m, still more preferably from 0.09 to 7.5 m/m, still more preferably from 0.1 to 7.25 m/m, still more preferably from 0.11 to 7.00 m/m, still more preferably from 0.12 to 6.75 m/m, still more preferably from 0.13 to 6.50 m/m, still more preferably from 0.14 to 6.25 m/m, even more preferably from 0.15 to 6.00 m/m, even more preferably from 0.16 to 5.75 m/m, even more preferably from 0.17 to 5.50 m/m, even more preferably from 0.18 to 5.25 m/m, even more preferably from 0.19 to 5.5 m/m, even more preferably from 0.20 to 5 m/m, even more preferably from 0.20 to 4.5 m/m, even more preferably from 0.20 to 4 m/m, even more preferably from 0.20 to 3.5 m/m, even more preferably from 0.20 to 3 m/m, even more preferably from 0.20 to 2.5 m/m, and even more preferably from 0.20 to 2 m/m.

The amount of the disintegrant can vary from 0.1 wt% to 40 wt%, preferably from 1 wt% to 35 wt%, even more preferably from 2 wt% to 30 wt%, even more preferably from 2.5 wt% to 29 wt%, even more preferably from 3.0 wt% to 28 wt%, even more preferably from 3.5 wt% to 27 wt%, and most preferably from 3.5 wt% to 26.5 wt%.

In addition, the amount of the disintegrant can vary from 0.1 mg to 150 mg, preferably from 0.50 mg to 145 mg, more preferably from 0.75 mg to 140 mg, still more preferably from 1.00 mg to 135 mg, still more preferably from 1.25 mg to 130 mg, still more preferably from 1.50 mg to 125 mg, still more preferably from 1.75 mg to 120 mg, still more preferably from 2.00 mg to 115 mg, still more preferably from 2.25 mg to 110 mg, still more preferably from 2.50 mg to 105 mg, still more preferably from 2.75 mg to 100 mg, still more preferably from 3.00 mg to 95 mg, even more preferably from 3.25 mg to 90 mg, even more preferably from 3.50 mg to 85 mg, even more preferably from 3.75 mg to 80 mg, even more preferably from 4.00 mg to 75 mg, even more preferably from 4.25 mg to 70 mg, even more preferably 4.50 mg to 65 mg, even more preferably 4.75 mg to 60 mg, even more preferably 5.00 mg to 55 mg, even more preferably 5.50 mg to 50 mg, even more preferably 6.00 mg to 45 mg, even more preferably 6.50 mg to 42.5 mg, even more preferably 7.00 mg to 40 mg, even more preferably 7.50 mg to 40 mg, even more preferably 8.00 mg to 40 mg, even more preferably 8.50 mg to 40 mg, even more preferably 9.00 mg to 40 mg, even more preferably 9.50 mg to 40 mg, and most preferably form 10 mg to 40 mg.

Furthermore, a mass ratio of the disintegrant relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof can range from 0.05 to 12 m/m, preferably from 0.06 to 11.5 m/m, more preferably from 0.07 to 11 m/m, still more preferably from 0.08 to 10.5 m/m, still more preferably from 0.09 to 10 m/m, still more preferably from 0.1 to 9.5 m/m, still more preferably from 0.11 to 9 m/m, still more preferably from 0.12 to 8.5 m/m, still more preferably from 0.13 to 8 m/m, still more preferably from 0.14 to 7.5 m/m, still more preferably from 0.15 to 7 m/m, even more preferably from 0.16 to 6.5 m/m, even more preferably from 0.17 to 5.5 m/m, even more preferably from 0.18 to 5 m/m, even more preferably from 0.19 to 5 m/m, and most preferably 0.2 to 5 m/m.

The amount of the lubricant/glidant can range from 0.1 wt% to 10 wt%, preferably from, more preferably from 0.25 wt% to 9.5 wt%, still more preferably from 0.5 wt% to 9 wt%, still more preferably from 0.75 wt% to 8.5 wt%, still more preferably from 1 wt% to 8 wt%, still more preferably from 1.25 wt% to 7.5 wt%, still more preferably from 1.5 wt% to 7 wt%, and even more preferably 1.5 wt% to 6.5 wt%.

Moreover, the amount of the lubricant/glidant can range from 0.01 mg to 100 mg, preferably from 0.05 mg to 95 mg, more preferably from 0.1 mg to 90 mg, still more preferably from 0.3mg to 85 mg, still more preferably from 0.4 mg to 80 mg, still more preferably from 0.5 mg to 0.6 mg, still more preferably from 0.7 mg to 70 mg, still more preferably from 0.8 mg to 65 mg, still more preferably from 0.9 mg to 60 mg, still more preferably from 1 mg to 55 mg, still more preferably from 1.1 mg to 50 mg, still more preferably from 1.2 mg to 45 mg, still more preferably from 1.3 mg to 40 mg, still more preferably from 1.4 mg to 35 mg, still more preferably from 1.5 mg to 30 mg, still more preferably from 1.6 mg to 25 mg, still more preferably from 1.7 mg to 20 mg, even more preferably from 1.8 mg to 20 mg, even more preferably from 1.9 mg to 20 mg, even preferably from 2 mg to 20 mg, even preferably from 3 mg to 20 mg, even preferably from 4 mg to 20 mg, and most preferably even preferably from 5 mg to 20 mg.

Furthermore, a mass ratio of the lubricant/glidant relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof can range from 0.05 to 2 m/m, preferably from 0.06 to 1.8 m/m, more preferably from 0.07 to 1.6 m/m, 0.08 to 1.4 m/m, still more preferably from 0.09 to 1.3 m/m, and most preferably from 0.1 to 1.2 m/m.

The amount of diluent/filler/binder in the composition can range from 0 wt% to 50% wt%, preferably from 1 wt% to 47.5% wt%, more preferred from 1.5 wt% to 45% wt%, more preferred from 2 wt% to 42.5% wt%, more preferred from 2.5 to 40 wt%, more preferred from 3 wt% to 38% wt%, more preferred 3.5 wt% to 38 wt%, more preferred 4 wt% to 38 wt%, more preferred to wt%, more preferred 4.5 wt% to 38 wt%, and even more preferred 5 wt% to 38 wt%

Moreover, the amount of the diluent/filler/binder can range from 1 mg to 290 mg, preferably from 2 mg to 280 mg, more preferably from 3 mg to 270 mg, even more preferably from 4 mg to 260 mg, even more preferably from 5 mg to 250 mg, even more preferably from 6 mg to 240 mg, even more preferably from 7 mg to 230 mg, even more preferably from 8 mg to 220 mg, even more preferably from 9 mg to 210 mg, even more preferably from 10 mg to 200 mg, even more preferably from 11 mg to 190 mg, even more preferably from 12 mg to 180 mg, even more preferably from 13 mg to 170 mg, even more preferably from 14 mg to 160 mg, even more preferably from 15 mg to 150 mg, even more preferably from 16 mg to 140 mg, even more preferably from 17 mg to 130 mg, even more preferably from 18 mg to 120 mg, even more preferably from 19 mg to 110 mg, even more preferably from 19 mg to 100 mg, even more preferably from 20 mg to 90 mg, more preferably from 21 mg to 80 mg, even more preferably from 22 mg to 70 mg, even more preferably from 23 mg to 60 mg, even more preferably from 24 mg to 55 mg, and most preferably 25 mg to 50 mg.

Furthermore, a mass ratio of the diluent/filler/binder relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof can range from 0 m/m to 20 m/m, more preferably 0.01 m/m to 17.5 m/m, more preferably 0.05 m/m to 15 m/m, more preferably 0.1 m/m to 0.125 m/m, more preferably 0.15 m/m to 10 m/m, more preferably 0.175 m/m to 7.5 m/m, more preferably 0.2 m/m to 6, more preferably 0.2 m/m to 5.5, more preferably 0.2 m/m to 5 m/m.

The amount of other ingredients can range from 5 wt% to 60 wt%, preferably from 6 wt% to 57.5 wt%, more preferably 7 wt% to 55 wt%, even more preferably from 8 wt% to 52.5 wt%, even more preferably from 9 wt% to 51 wt%, and most preferably 10 wt% to 50 wt% with respect to dosage form.

In addition, the amount of other ingredients can range from 50 mg to 200 mg, preferably from 55 mg to 190 mg, more preferably from 60 mg to 180 mg, still more preferably from 65 mg to 170 mg, still more preferably from 70 mg to 160 mg, still more preferably from 75 mg to 150 mg, still more preferably from 80 mg to 140 mg, still more preferably from 90 mg to 130 mg, even more preferably from 90 mg to 120 mg, even more preferably from 90 mg to 110 mg, and most preferably from 90 mg to 100 mg.

Furthermore, a mass ratio of other ingredients relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof can range from 0 to 30 m/m, preferably from 0.2 to 27.5 m/m, more preferably from 0.3 to 25 m/m, still more preferably from 0.35 to 22.5 m/m, even more preferably from 0.4 to 21 m/m, and most preferably from 0.45 to 20 m/m.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 1 wt% to 75 wt% acidifier.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3 wt% to 75 wt% acidifier.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 4.5 wt% to 55 wt% acidifier.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 1 wt% to 75 wt% acidifier.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3 wt% to 75 wt% acidifier.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 4.5 wt% to 55 wt% acidifier.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 1 wt% to 75 wt% acidifier.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3 wt% to 75 wt% acidifier.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 4.5 wt% to 55 wt% acidifier.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 1 wt% to 75 wt% acidifier.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3 wt% to 75 wt% acidifier.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 4.5 wt% to 55 wt% acidifier.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 1 wt% to 75 wt% adipic acid.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3 wt% to 75 wt% adipic acid.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 4.5 wt% to 55 wt% adipic acid.

A more preferred embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 1 wt% to 75 wt% adipic acid.

A more preferred embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3 wt% to 75 wt% adipic acid.

A more preferred embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 4.5 wt% to 55 wt% adipic acid.

A preferred embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 1 wt% to 75 wt% adipic acid.

A preferred embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3 wt% to 75 wt% adipic acid.

A preferred embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 4.5 wt% to 55 wt% adipic acid.

A preferred embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 1 wt% to 75 wt% adipic acid.

A preferred embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3 wt% to 75 wt% adipic acid.

A preferred embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 4.5 wt% to 55 wt% adipic acid.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 wt% to 40 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 2 wt% to 35 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3.5 wt% to 30 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 wt% to 40 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3.5 wt% to 35 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 2 wt% to 30 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 wt% to 40 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 2 wt% to 35 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3.5 wt% to 30 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 wt% to 40 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 2 wt% to 35 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3.5 wt% to 30 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 wt% to 40 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 2 wt% to 35 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3.5 wt% to 30 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 wt% to 40 wt% L-hydroxypropyl cellulose and/or hydroxypropyl cellulose.

A preferred embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 2 wt% to 35 wt% L-hydroxypropyl cellulose and/or hydroxypropyl cellulose.

A preferred embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3.5 wt% to 30 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 wt% to 40 wt% L-hydroxypropyl cellulose and/or hydroxypropyl cellulose.

A preferred embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 2 wt% to 35 wt% L-hydroxypropyl cellulose and/or hydroxypropyl cellulose.

A preferred embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3.5 wt% to 30 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 wt% to 40 wt% L-hydroxypropyl cellulose and/or hydroxypropyl cellulose.

A preferred embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 2 wt% to 35 wt% L-hydroxypropyl cellulose and/or hydroxypropyl cellulose.

A preferred embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 3.5 wt% to 30 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 0.1 wt% to 40 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 2 wt% to 35 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 3.5 wt% to 30 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 0.1 wt% to 40 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 2 wt% to 35 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 3.5 wt% to 30 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 0.1 wt% to 40 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 2 wt% to 35 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 3.5 wt% to 30 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 0.1 wt% to 40 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 2 wt% to 35 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 3.5 wt% to 30 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 0.1 wt% to 40 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 2 wt% to 35 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 3.5 wt% to 30 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 0.1 wt% to 40 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 2 wt% to 35 wt% polymeric precipitation inhibitor.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 3.5 wt% to 30 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 0.1 wt% to 40 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 2 wt% to 35 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 3.5 wt% to 30 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 0.1 wt% to 40 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 2 wt% to 35 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 3.5 wt% to 30 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 0.1 wt% to 40 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 2 wt% to 35 wt% polymeric precipitation inhibitor.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 3.5 wt% to 30 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 0.1 wt% to 40 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 2 wt% to 35 wt% polymeric precipitation inhibitor.

An embodiment of the invention is related to a systemic formulation comprising or consisting of 0.1 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, and 3.5 wt% to 30 wt% polymeric precipitation inhibitor.

An embodiment of the invention is related to a systemic formulation comprising or consisting of 0.1 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 0.1 wt% to 40 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 2 wt% to 35 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, and 3.5 wt% to 30 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 0.1 wt% to 40 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 2 wt% to 35 wt% polymeric precipitation inhibitor.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, and 3.5 wt% to 30 wt% polymeric precipitation inhibitor.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 0.1 wt% to 40 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 2 wt% to 35 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 3.5 wt% to 30.5 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 0.1 wt% to 40 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 2 wt% to 35 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 3.5 wt% to 30.5 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 0.1 wt% to 40 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1 ,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 2 wt% to 35 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 3.5 wt% to 30.5 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 0.1 wt% to 40 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 2 wt% to 35 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 3.5 wt% to 30.5 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 0.1 wt% to 40 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 2 wt% to 35 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 3.5 wt% to 30.5 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 0.1 wt% to 40 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 2 wt% to 35 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 3.5 wt% to 30.5 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 0.1 wt% to 40 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 2 wt% to 35 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 3.5 wt% to 30.5 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 0.1 wt% to 40 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 2 wt% to 35 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 3.5 wt% to 30.5 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 0.1 wt% to 40 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 2 wt% to 35 wt% L-hydroxypropyl cellulose and/or hydroxypropyl cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 3.5 wt% to 30.5 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 0.1 wt% to 40 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 2 wt% to 35 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 3.5 wt% to 30.5 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 0.1 wt% to 40 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 2 wt% to 35 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, and 3.5 wt% to 30.5 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 0.1 wt% to 40 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 2 wt% to 35 wt% L-hydroxypropyl cellulose and/or hydroxypropyl cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, and 3.5 wt% to 30.5 wt% L-hydroxypropyl cellulose and/or hydroxypropyl cellulose.

An embodiment of the invention is related to a systemic formulation comprising or consisting of (S,E)-methyl-7-(1-(2-(2-ethylbutylamino)-2-oxoethyl)-2-oxo-1 ,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, and 0 wt% to 15 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, and 0 wt% to 12 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, and 0 wt% to 12 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, and 0 wt% to 12 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, and 0 wt% to 12 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, and 0 wt% to 12 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, and 0 wt% to 12 wt% binder

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, and 0 wt% to 12 wt% binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, and 0 wt% to 12 wt% binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, and 0 wt% to 12 wt% binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, and 0 wt% to 12 wt% binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, and 0 wt% to 12 wt% binder.

An embodiment of the invention is related to a systemic formulation comprising or consisting of 0.1 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of 0.1 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, and 0 wt% to 12 wt% binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, and 0 wt% to 12 wt% binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, and 0 wt% to 12 wt% binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, and 0 wt% to 12 wt% binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, and 0 wt% to 12 wt% binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, and 0 wt % to 15 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 2 wt% to 35 wt% L-hydroxypropyl cellulose and/or hydroxypropyl cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 15 wt% binder, 0.1 wt% to 35 wt% disintegrant, and 0.1 wt% to 10 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, 0 wt% to 12 wt% binder,2 wt% to 35 wt% disintegrant, and 1 wt% to 9 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant,2 wt% to 35 wt% disintegrant, and 1 wt% to 9 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, 2 wt% to 35 wt% disintegrant, and 1 wt% to 9 wt% lubricant/glidant..

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, 2 wt% to 35 wt% disintegrant, and 1 wt% to 9 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

A preferred embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1 -methyl-1 H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

A very preferred embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt% to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 0.1 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, and 0 wt% to 12 wt% binder.

An embodiment of the invention is related to a systemic formulation comprising or consisting of 0.1 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 15 wt% povidone K25, 0.1 wt% to 35 wt% sodium croscarmellose, and 0.1 wt% to 10 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, 2 wt% to 35 wt% sodium croscarmellose, and 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, and 0 wt % to 12 wt% povidone K25.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, and 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, 0 wt % to 12 wt% povidone K25, , 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30 wt% L-hydroxypropyl cellulose and/or hydroxypropyl cellulose, 0 wt % to 12 wt% povidone K25, 2 wt% to 35 wt% sodium croscarmellose, and 1 wt% to 9 wt% talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 15 wt% binder, 0.1 wt% to 35 wt% disintegrant, 0.1 wt% to 10 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, 0 wt% to 12 wt% binder,2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant,2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 0.1 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 3.5 wt% to 30 wt% polymeric precipitation inhibitor, 0 wt% to 12 wt% binder, and 0 wt% to 50 wt% diluent/filler/binder.

An embodiment of the invention is related to a systemic formulation comprising or consisting of 0.1 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0.1 wt% to 40 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 2 wt% to 35 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 3.5 wt% to 30 wt% polymeric precipitation inhibitor, 0 wt% to 12 wt% binder, 2 wt% to 35 wt% disintegrant, 1 wt% to 9 wt% lubricant/glidant, and 0 wt% to 50 wt% diluent/filler/binder.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 15 wt% povidone K25, 0.1 wt% to 35 wt% sodium croscarmellose, 0.1 wt% to 10 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt% to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, 0 wt % to 12 wt% povidone K25, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 0.1 wt% to 40 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 0.1 wt% to 40 wt% L-hydroxypropyl cellulose and/or hydroxypropyl cellulose, 0 wt % to 12 wt% povidone K25, , 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 2 wt% to 35 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% adipic acid, 3.5 wt% to 30.5 wt% L-hydroxypropyl cellulose and/or hydroxypropyl cellulose, 0 wt % to 12 wt% povidone K25, 2 wt% to 35 wt% sodium croscarmellose, 1 wt% to 9 wt% talc or silicon dioxide, and 0 wt% to 50 wt% mannitol and/or microcrystalline cellulose.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0 to 50 wt% diluent/filler/binder, 0.1 wt% to 40 wt% polymeric precipitation inhibitor, 0 wt% to 15 wt% binder, 0.1 wt% to 35 wt% disintegrant, and 0.1 wt% to 10 wt% lubricant/glidant.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 wt% to 75 wt% acidifier, 0 to 50 wt% diluent/filler/binder, 0.1 wt% to 40 wt% polymeric precipitation inhibitor, 0 wt% to 12 wt% binder, 2 wt% to 35 wt% disintegrant, and 1 wt% to 9 wt% lubricant/glidant.

A more preferred embodiment according to the invention is directed to a systemic formulation comprising or consisting of of 0.1 wt% to 35 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0 wt% to 50 wt% diluent/filler/binder, 1 wt% to 75 wt% acidifier, 2 wt% to 25 wt% polymeric precipitation inhibitor, 0 wt% to 12 wt% binder, 2 wt% to 35 wt% disintegrant, and 1 wt% to 9 wt% lubricant/glidant.

A more preferred embodiment according to the invention is directed to a systemic formulation comprising or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0 wt% to 50 wt% diluent/filler/binder, 1 wt% to 75 wt% acidifier, 2 wt% to 25 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 3 wt% to 75 wt% acidifier, 0 to 50 wt% diluent/filler/binder, 0.1 wt% to 40 wt% polymeric precipitation inhibitor, 0 wt% to 12 wt% binder, 2 wt% to 35 wt% disintegrant, and 1 wt% to 9 wt% lubricant/glidant.

A more preferred embodiment according to the invention is directed to a systemic formulation comprising or consisting of or consisting of 0.1 wt% to 35 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0 wt% to 50 wt% diluent/filler/binder, 3 wt% to 75 wt% acidifier, 2 wt% to 25 wt% polymeric precipitation inhibitor, 0 wt% to 12 wt% binder, 2 wt% to 35 wt% disintegrant, and 1 wt% to 9 wt% lubricant/glidant.

A more preferred embodiment according to the invention is directed to a systemic formulation comprising or consisting of or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0 wt% to 50 wt% diluent/filler/binder, 3 wt% to 75 wt% acidifier, 2 wt% to 25 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 4.5 wt% to 55 wt% acidifier, 0 to 50 wt% diluent/filler/binder, 0.1 wt% to 40 wt% polymeric precipitation inhibitor, 0 wt% to 12 wt% binder, 2 wt% to 35 wt% disintegrant, and 1 wt% to 9 wt% lubricant/glidant.

A more preferred embodiment according to the invention is directed to a systemic formulation comprising or consisting of or consisting of 0.1 wt% to 35 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0 wt% to 50 wt% diluent/filler/binder, 4.5 wt% to 55 wt% acidifier, 2 wt% to 25 wt% polymeric precipitation inhibitor, 0 wt% to 12 wt% binder, 2 wt% to 35 wt% disintegrant, and 1 wt% to 9 wt% lubricant/glidant.

A more preferred embodiment according to the invention is directed to a systemic formulation comprising or consisting of or consisting of 2.5 wt% to 30.5 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0 wt% to 50 wt% diluent/filler/binder, 4.5 wt% to 55 wt% acidifier, 2 wt% to 25 wt% polymeric precipitation inhibitor, 0 wt% to 12 wt% binder, 2 wt% to 35 wt% disintegrant, and 1 wt% to 9 wt% lubricant/glidant.

An embodiment of the invention is related to a systemic formulation comprising or consisting of or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoat or an enantiomer, a solvate, hydrate or a pharmaceutically acceptable salt thereof, 0 to 50 wt% microcrystalline cellulose, 5 wt% to 75 wt% adipic acid, 0.1 wt% to 40 wt% L-hydroxypropyl cellulose and/or hydroxypropyl cellulose, 0 wt% to 15 wt%povidone K25, 0.1 wt% to 35 wt% sodium croscarmellose, and 0.1 wt% to 9 wt% talc or silicon dioxide.

An embodiment of the invention is related to a systemic formulation comprising or consisting of 0.1 wt% to 80 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoat or an enantiomer, a solvate, hydrate or a pharmaceutically acceptable salt thereof, 0 to 50 wt% microcrystalline cellulose, 1 wt% to 75 wt% adipic acid, 0.1 wt% to 40 wt% L-hydroxypropyl cellulose and/or hydroxypropyl cellulose, 0 wt% to 15 wt%povidone K25, 2 wt% to 35 wt% sodium croscarmellose, and 1 wt% to 9 wt% talc or silicon dioxide.

A more preferred embodiment according to the invention is directed to a systemic formulation comprising or consisting of 0.1 wt% to 35 wt% (S,E)-methyl-7-(1-(2-(2-ethylbutylamino)-2-oxoethyl)-2-oxo-1,2-dihydro-pyridin-3-ylamino)-6-(1 -methyl-1H-imidazole-5-carboxamido)-7-oxohept-2-enoate or an enantiomer, a solvate, hydrate or a pharmaceutically acceptable salt thereof, 0 to 50 wt% microcrystalline cellulose, 1 wt% to 75 wt% adipic acid, 0.1 wt% to 40 wt% L-hydroxypropyl cellulose and/or hydroxypropyl cellulose, 0 wt% to 15 wt% povidone K25, 2 wt% to 35 wt% sodium croscarmellose, and 1 wt% to 9 wt% talc or silicon dioxide.

If the systemic formulation is discloses as a formulation consisting of ingredients in certain amounts (weight percent, mass ratio and/or absolute mass), the rest are other ingredients, i.e. it is balanced to 100 wt% with other ingredients. The other ingredients are described above.

An embodiment of the invention is related to a systemic formulation for use in the prophylaxis and/or treatment of the diseases disclosed herein comprising or consisting of at least 0.01 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 500 mg adipic acid, and 1 mg to 100 mg L-hydroxypropyl cellulose.

A preferred embodiment of the invention is related to a systemic formulation comprising or consisting of 0.01 mg to 500 mg (S,E)-methyl-7-(1-(2-(2-ethylbutylamino)-2-oxoethyl)-2-oxo-1,2-dihydro-pyridin-3-ylamino)-6-(1 -methyl-1H-imidazole-5-carboxamido)-7-oxohept-2-enoate or an enantiomer, a solvate, hydrate or a pharmaceutically acceptable salt thereof, 1 mg to 500 mg adipic acid, and 1 mg to 100 mg L-hydroxypropyl cellulose and/or hydroxypropyl cellulose, and up to 100 mg povidone K25.

An embodiment of the invention is related to a systemic formulation comprising or consisting of 0.01 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 500 mg adipic acid, 1 mg to 100 mg L-hydroxypropyl cellulose and hydroxypropyl cellulose, up to 100 mg povidone K25, 1 mg to 100 mg sodium croscarmellose, and 1 mg to 50 mg talc or silicon dioxide.

An embodiment of the invention is related to a systemic formulation comprising or consisting of 0.01 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 360 mg adipic acid, 10 mg to 40 mg L-hydroxypropyl cellulose, 10 mg to 40 mg sodium croscarmellose, and 1 mg to 20 mg talc or silicon dioxide.

An embodiment of the invention is related to a systemic formulation comprising or consisting of at least 0.1 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 500 mg adipic acid, and 1 mg to 100 mg L-hydroxypropyl cellulose.

A preferred embodiment of the invention is related to a systemic formulation comprising or consisting of 0.1 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 500 mg adipic acid, and 1 mg to 100 mg L-hydroxypropyl cellulose and/or hydroxypropyl cellulose, and up to 100 mg povidone K25.

An embodiment of the invention is related to a systemic formulation comprising or consisting of 0.1 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 500 mg adipic acid, 1 mg to 100 mg L-hydroxypropyl cellulose and hydroxypropyl cellulose, up to 100 mg povidone K25, 1 mg to 100 mg sodium croscarmellose, and 1 mg to 50 mg talc or silicon dioxide.

An embodiment of the invention is related to a systemic formulation comprising or consisting of 0.1 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 360 mg adipic acid, 10 mg to 40 mg L-hydroxypropyl cellulose, 10 mg to 40 mg sodium croscarmellose, and 1 mg to 20 mg talc or silicon dioxide.

A preferred embodiment of the invention is related to a systemic formulation comprising or consisting of 0.5 mg to 500 mg (S,E)-methyl-7-(1-(2-(2-ethylbutylamino)-2-oxoethyl)-2-oxo-1,2-dihydro-pyridin-3-ylamino)-6-(1 -methyl-1H-imidazole-5-carboxamido)-7-oxohept-2-enoate or an enantiomer, a solvate, hydrate or a pharmaceutically acceptable salt thereof, 1 mg to 500 mg adipic acid, and 1 mg to 100 mg L-hydroxypropyl cellulose and/or hydroxypropyl cellulose, and up to 100 mg povidone K25.

An embodiment of the invention is related to a systemic formulation comprising or consisting of 0.5 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 500 mg adipic acid, 1 mg to 100 mg L-hydroxypropyl cellulose and hydroxypropyl cellulose, up to 100 mg povidone K25, 1 mg to 100 mg sodium croscarmellose, and 1 mg to 50 mg talc or silicon dioxide.

An embodiment of the invention is related to a systemic formulation comprising or consisting of 0.5 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 360 mg adipic acid, 10 mg to 40 mg L-hydroxypropyl cellulose, 10 mg to 40 mg sodium croscarmellose, and 1 mg to 20 mg talc or silicon dioxide.

A preferred embodiment of the invention is related to a systemic formulation comprising or consisting of 1 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 500 mg adipic acid, and 1 mg to 100 mg L-hydroxypropyl cellulose and/or hydroxypropyl cellulose, and up to 100 mg povidone K25.

An embodiment of the invention is related to a systemic formulation comprising or consisting of 1 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 500 mg adipic acid, 1 mg to 100 mg L-hydroxypropyl cellulose and hydroxypropyl cellulose, up to 100 mg povidone K25, 1 mg to 100 mg sodium croscarmellose, and 1 mg to 50 mg talc or silicon dioxide.

An embodiment of the invention is related to a systemic formulation comprising or consisting of 1 mg to 500 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 1 mg to 360 mg adipic acid, 10 mg to 40 mg L-hydroxypropyl cellulose, 10 mg to 40 mg sodium croscarmellose, and 1 mg to 20 mg talc or silicon dioxide.

An embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, and 15 to 1 m/m acidifier, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 11.5 to 1.5 m/m acidifier, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1 -methyl-1H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 11.5 to 1.8 m/m acidifier, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1 -methyl-1H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.1 to 7 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and 0.2 to 5 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m acidifier, and 0.1 to 7 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.5 m/m acidifier, and 0.1 to 7 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, and 0.1 to 7 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m acidifier, and 0.2 to 5 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.5 m/m acidifier, and 0.2 to 5 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, and 0.2 to 5 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m acidifier, 0.1 to 7 m/m polymeric precipitation inhibitor, 0 to 5 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.5 m/m acidifier, 0.1 to 7 m/m polymeric precipitation inhibitor, 0 to 5 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.1 to 7 m/m polymeric precipitation inhibitor, 0 to 5 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m acidifier, 0.2 to 5 m/m polymeric precipitation inhibitor, 0 to 5 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.5 m/m acidifier, 0.2 to 5 m/m polymeric precipitation inhibitor, 0 to 5 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.2 to 5 m/m polymeric precipitation inhibitor, 0 to 5 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m acidifier, 0.1 to 7 m/m polymeric precipitation inhibitor, 0 to 2 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.5 m/m acidifier, 0.1 to 7 m/m polymeric precipitation inhibitor, 0 to 2 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.1 to 7 m/m polymeric precipitation inhibitor, 0 to 2 m/m binder, 0.1 to 7 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, 15 to 1 m/m acidifier, 0.1 to 7 m/m polymeric precipitation inhibitor, 0 to 5 m/m binder, 0.1 to 7 m/m disintegrant, and 0.1 to 1.5 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.5 m/m acidifier, 0.1 to 7 m/m polymeric precipitation inhibitor, 0 to 5 m/m binder, 0.1 to 7 m/m disintegrant, and 0.1 to 1.5 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.1 to 7 m/m polymeric precipitation inhibitor, 0 to 5 m/m binder, 0.1 to 7 m/m disintegrant, and 0.1 to 1.5 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m acidifier, 0.1 to 7 m/m polymeric precipitation inhibitor, 0 to 5 m/m binder, 0.2 to 5 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.5 m/m acidifier, 0.1 to 7 m/m polymeric precipitation inhibitor, 0 to 5 m/m binder, 0.2 to 5 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.1 to 7 m/m polymeric precipitation inhibitor, 0 to 5 m/m binder, 0.2 to 5 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m acidifier, 0.1 to 7 m/m polymeric precipitation inhibitor, 0 to 5 m/m binder, 0.1 to 7 m/m disintegrant, 0.05 to 2 m/m lubricant/glidant, 0 to 5 m/m diluent/filler/binder, and 0 m/m to 25 m/m other ingredients, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.2 to 5 m/m polymeric precipitation inhibitor, 0 to 2 m/m binder, 0.2 to 5 m/m disintegrant, and 0.05 to 2 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.2 to 5 m/m polymeric precipitation inhibitor, 0 to 2 m/m binder, 0.2 to 5 m/m disintegrant, 0.05 to 2 m/m lubricant/glidant, and 0 to 5 m/m diluent/filler/binder, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1 -methyl-1H-imidazole-5-carboxamido)-7-oxohept-2-enoate or an enantiomer, a solvate, hydrate or a pharmaceutically acceptable salt thereof.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.2 to 5 m/m polymeric precipitation inhibitor, 0 to 2 m/m binder, 0.2 to 5 m/m disintegrant, 0.05 to 2 m/m lubricant/glidant, 0 to 5 m/m diluent/filler/binder, and 0 m/m to 19.5 m/m other ingredients, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

A particularly preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m acidifier, 0.2 to 5 m/m polymeric precipitation inhibitor, 11.5 to 1.5 m/m binder, 0.2 to 5 m/m disintegrant, and 0.1 to 1.5 m/m lubricant/glidant, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

Another preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m adipic acid, and 0.1 to 7 m/m L-hydroxypropyl cellulose and/or hydroxypropyl cellulose, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

Another particularly preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m adipic acid, and 0.2 to 5 m/m L-hydroxypropyl cellulose and/or hydroxypropyl cellulose, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

Another preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 15 to 1 m/m adipic acid, 0.1 to 7 m/m L-hydroxypropyl cellulose and/or hydroxypropyl cellulose, 0 to 2 m/m povidone K25, 0.2 to 5 m/m sodium croscarmellose, and 0.05 to 2 m/m talc or silicon dioxide, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

Another particularly preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 11.5 to 1.8 m/m adipic acid, 0.2 to 5 m/m L-hydroxypropyl cellulose and/or hydroxypropyl cellulose, 0 to 1.5 m/m povidone K25, 0.2 to 5 m/m sodium croscarmellose, and 0.1 to 1.2 m/m talc or silicon dioxide, wherein m/m (mass ratio) of said compounds is calculated relative to mass of (S,E)-methyl-7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate or an enantiomer, a solvate, hydrate or a pharmaceutically acceptable salt thereof.

A mass ratio of the acidifier relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof ranges from 11.5 to 1 m/m, and a mass ratio of the polymeric precipitation inhibitor relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof ranges from 0.2 to 5 m/m is preferred.

More preferred is a mass ratio of the acidifier relative to the mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof ranges from 11.5 to 1.8 m/m, and a mass ratio of the polymeric precipitation inhibitor relative to the mass of (S,E)-methyl-7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate or an enantiomer, a solvate, hydrate or a pharmaceutically acceptable salt thereof ranges from 0.2 to 5 m/m.

A mass ratio between the acidifier and the polymeric precipitation inhibitor can vary from 0.01 m/m to 20 m/m, preferably from 0.02 m/m to 15 m/m, more preferably from 0.03 m/m to 12.5 m/m, even more preferably from 0.04 m/m to 10 m/m, even more preferably from 0.05 m/m, even more preferably from 0.06 m/m to 9 m/m, even more preferably from 0.07 m/m to 8 m/m, even more preferably from 0.08 m/m to 7 m/m, even more preferably from 0.09 m/m to 6 m/m, even more preferably from 0.1 m/m to 5 m/m, even more preferably from 0.1 m/m to 4.5 m/m, even more preferably from 0.1 m/m to 4 m/m, even more preferably from 0.1 m/m to 3.5 m/m, and most preferably from 0.1 m/m to 3 m/m, wherein the mass ration is calculated relative to the mass of the acidifier in the formulation.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, acidifier, and polymeric precipitation inhibitor, wherein the mass ratio between the solution stabilzer and the acidifier ranges from 0.01 to 6 m/m, and wherein m/m (mass ratio) of said compounds is calculated relative to the mass of the acidifier.
Preferably, the formulation according to the invention is not included in a pump which is implantable. The administration by an osmotic pump is not applicable to human.

A preferred embodiment of the invention is related to a systemic formulation comprising or consisting of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, acidifier, and polymeric precipitation inhibitor, wherein the mass ratio between the solution stabilzer and the acidifier ranges from 0.05 to 3.5 m/m, and wherein m/m (mass ratio) of said compounds is calculated relative to the mass of the acidifier.
Preferably, the formulation according to the invention is not included in a pump which is implantable. The administration by an osmotic pump is not applicable to human.

Preferably, the maximal concentration in the plasma or tissue (Cmax-value) of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof after adminstration is 0.2 ng/mL to 2000 ng/mL, and wherein the Cmax-value is measured in plasma samples by liquid chromatography coupled with mass spectrometry.

The inhibitory effect is dependent from the maximal plasma or tissue concentration (cmax-value).

Another aspect of the invention is directed to a formulation according to the invention for use as a medicine preferably in mammal, and most preferably in human.

A preferred embodiment of the invention is related to a systemic formulation for use in the prophylaxis and/or treatment, wherein the TG2-related diseases. Preferably the TG2-related diseases are selected from the group comprising or consisting of nephropathy, fibrotic liver diseases including NAFLD, NASH, cirrhosis, cholestatic liver diseases such as primary sclerosing cholangitis (PSC), and primary biliary cholangitis (PBC), autoimmune hepatitis (AIH), alcoholic steatohepatitis (ASH), cystic fibrosis, pulmonary fibrosis, idiopathic pulmonary fibrosis, radiation-induced lung injury, bridging fibrosis, cardiac fibrosis, systemic sclerosis, collagen induced arthritis (CIA), rheumatoid arthritis (RA), atrial fibrosis, endomyocardial fibrosis, old myocardial infarction, vascular stiffening, vascular calcification, fibroproliferative diseases, elevated blood pressure, gliar scar, arterial stiffness, arthrofibrosis, Dupuytren's contracture, keloid, medistinal fibrosis, myelofibrosis, Peyronie's disease, nephrongenic systemic fibrosis, progressive massive fibrosis, retroperitoneal fibrosis, systemic sclerosis, and adhersisve capsulitis preferably in human. Most preferably the TG2-related disease is selected from nephropathy, NASH, cystic fibrosis, and diabetes related fibrosis.

A more preferred embodiment of the invention is related to a systemic formulation for use in the prophylaxis and/or treatment, wherein the TG2-related disease is selected from the group comprising or consisting of nephropathy, fibrotic liver diseases including NAFLD, NASH, cirrhosis, cholestatic liver diseases such as primary sclerosing cholangitis (PSC), and primary biliary cholangitis (PBC), autoimmune hepatitis (AIH), alcoholic steatohepatitis (ASH) and/or cystic fibrosis, pulmonary fibrosis, idiophatic pulmonary fibrosis, cardiac fibrosis, systemic sclerosis, collagen induced arthritis (CIA), rheumatoid arthritis (RA), vascular stiffening, vascular calcification, fibroproliferative diseases, and elevated blood pressure preferably in human.

A preferred embodiment according to the invention is related to a systemic formulation for use in the prophylaxis and/or in the treatment of fibrosis preferably in human.

Thus, a further aspect of the invention is related to a compound of formula (I) for use in the prophylaxis and/or in the treatment of fibrosis preferably in human. Another aspect of the invention is related to a salt of compound of formula (I) for use in the prophylaxis and/or in the treatment of fibrosis preferably in human.
Still another aspect of the invention is related to a salt of compound of formula (I) and adipic acid for use in the prophylaxis and/or in the treatment of fibrosis preferably in human.

The fibrosis is more preferably diabetes related fibrosis preferably in human. An especially preferred embodiment of the invention is thus directed to a formulation preferably systemic formulation, more preferably enteral formulation, and most preferably an oral formulation according to the invention for the prophylaxis and/or treatment of diabetic nephropathy, diabetic associated non-alcoholic steatohepatitis and/or cystic fibrosis related diabetes. Thus, in a preferred embodiment of the invention the TG2-related disease is selected from the group consisting of nephropathy, NASH and/or cystic fibrosis. In a further preferred embodiment of the invention, the TG2-related disease comprises or consist of diabetes related fibrosis.

An embodiment of the invention is related to a formulation according to the invention for use in the prophylaxis and/or treatment of a disease selected from the group comprising or consisting of nephropathy, NASH and/or cystic fibrosis.

Another aspect of the invention is directed to a method for preparation of a formulation according to the invention comprising the step:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

The amount of (S,E)-methyl 7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof provided in step A-1) is mentioned above.

Thus, an embodiment of the invention is directed to a method for preparation of a formulation according to the invention comprising the step:
A-1) Providing 1 mg to 500 g (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

Thus, an embodiment of the invention is directed to a method for preparation of a formulation according to the invention comprising the step:
A-1) Providing 1 mg to 500 g (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

Another embodiment of the invention is directed to a method for preparation of a formulation according to the invention comprising the step:
A-1) Providing 0.1 wt% to 80 wt% (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof.

It is apparent that the amount of the drug can also be substitute by another amount as mentioned above.

In step A-2), at least one excipient, as described herein, is added. Preferably, at least one excipient is a polymeric precipitation inhibitor preferably L-hydroxypropyl cellulose, a disintegrant or a diluent/filler/binder.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2) adding at least one excipient.

In a step A-2'), the (S,E)-methyl 7-(1-(2-(2-ethylbutylamino)-2-oxoethyl)-2-oxo-1,2-dihydro-pyridin-3-ylamino)-6-(1-methyl-1 H-imidazole-5-carboxamido)-7-oxohept-2-enoate or an enantiomer, a solvate, hydrate or a pharmaceutically acceptable salt thereof, a polymeric precipitation inhibitor preferably L-hydroxypropyl cellulose and a disintegrant preferably sodium croscarmellose are sieved in a dry state preferably separately.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') Sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, a polymeric precipitation inhibitor and a disintegrant in a dry state, preferably separately.

The amount of the disintegrant, polymeric precipitation inhibitor and disintegrant in step A-2') is mentioned above.

Thus, an embodiment according to the invention is related to a method for preparation of a formulation according to the invention comprising the steps:
A-1) Providing 1 mg to 500 mg (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') Sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 10 mg to 50 mg polymeric precipitation inhibitor and 10 mg to 40 mg disintegrant in a dry state, preferably separately.

A preferred embodiment according to the invention is related to a method for preparation of a formulation according to the invention comprising the steps:
A-1) Providing 1 mg to 500 mg (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') Sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 10 mg to 50 mg L-hydroxypropyl cellulose and 10 mg to 40 mg sodium croscarmellose in a dry state, preferably separately.

Another preferred embodiment according to the invention is related to a method for preparation of a formulation according to the invention comprising the steps:
A-1) Providing 0.1 wt% to 80 wt% (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') Sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0.1 wt% to 40 wt% polymeric precipitation inhibitor and 2 wt% to 15 wt% disintegrant in a dry state, preferably separately.

Another more preferred embodiment according to the invention is related to a method for preparation of a formulation according to the invention comprising the steps:
A-1) Providing 0.1 wt% to 80 wt% (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') Sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0.1 wt% to 40 wt% L-hydroxypropyl cellulose and/ povidone K25 and 2 wt% to 15 wt% sodium croscarmellose in a dry state, preferably separately.

It is apparent that the amount of the excipients and the drug can also be substitute by another amount as mentioned above.

In a step A-3), a solvent can be added which leads to a particle agglomeration and the formation of the granule structure. Preferably, the solvent is ethanol.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof,
A-2) adding at least one excipient, and
A-3) adding a solvent which leads to a particle agglomeration and the formation of the granule structure.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, a polymeric precipitation inhibitor and a disintegrant in a dry state, and
A-3) adding a solvent which leads to a particle agglomeration and the formation of the granule structure.

In a step A-4), the granule mass can be sieved in a wet state preferabyl the granule mass of step A-3).

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, a polymeric precipitation inhibitor and a disintegrant in a dry state,
A-3) adding a solvent which leads to a particle agglomeration and the formation of the granule structure, and
A-4) sieving the granule mass of step A-3) in a wet state.

In a step A-5), a granule mass in a wet state is dried, wherein dry granules are received.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, a polymeric precipitation inhibitor and a disintegrant in a dry state,
A-3) adding a solvent which leads to a particle agglomeration and the formation of the granulate structure,
A-4) sieving the granulate mass of step A-3) in a wet state, and
A-5) drying the granule mass in a wet state.

In a step B-1), an excipient preferably adipic acid and/or talc can be added to the dry granule, and mixed preferably in a dry mixer forming a powder mixture.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
B-1) adding at least one excipient.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof,
A-2) adding at least one excipient, and
A-3) adding a solvent which leads to a particle agglomeration and the formation of the granule structure.
B-1) adding at least one excipient.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, a polymeric precipitation inhibitor and a disintegrant in a dry state,
A-3) adding a solvent which leads to a particle agglomeration and the formation of the granulate structure,
A-4) sieving the granulate mass of step A-3) in a wet state,
A-5) drying the granule mass in a wet state, and
B-1) adding at least one excipient such as an acidifier preferably adipic acid and/or a lubricant/glidant preferably talc or silicon dioxide to the dry granule.

Thus, an embodiment according to the invention is related to a method for preparation of a formulation according to the invention comprising the steps:
A-1) Providing 1 mg to 500 mg (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 10 mg to 50 mg polymeric precipitation inhibitor and 10 mg to 40 mg disintegrant in a dry state,
A-3) adding a solvent which leads to a particle agglomeration and the formation of the granulate structure,
A-4) sieving the granulate mass of step A-3) in a wet state,
A-5) drying the granule mass in a wet state, and
B-1) adding 10 to 360 mg acidifier and/or 1 mg to 20 mg lubricant/glidant preferably talc or silicon dioxide to the dry granule.

Thus, an embodiment according to the invention is related to a method for preparation of a formulation according to the invention comprising the steps:
A-1) Providing 0.1 wt% to 80 wt% (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0.1 wt% to 40 wt% polymeric precipitation inhibitor and 2 wt% to 15 wt% disintegrant in a dry state,
A-3) adding a solvent which leads to a particle agglomeration and the formation of the granulate structure,
A-4) sieving the granulate mass of step A-3) in a wet state,
A-5) drying the granule mass in a wet state, and
B-1) adding 5 wt% to 75 wt% acidifier and/or 0.1 wt% to 10 wt% lubricant/glidant preferably talc or silicon dioxide to the dry granule.

A preferred embodiment according to the invention is related to a method for preparation of a formulation according to the invention comprising the steps:
A-1) Providing 1 mg to 500 mg (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 10 mg to 50 mg L-hydroxypropyl cellulose and/or povidone K25 and 10 mg to 40 mg sodium croscarmellose are sieved in a dry state preferably separately.
A-3) adding a solvent which leads to a particle agglomeration and the formation of the granulate structure,
A-4) sieving the granulate mass of step A-3) in a wet state,
A-5) drying the granule mass in a wet state, and
B-1) adding 10 to 360 mg adipic acid and 1 mg to 20 mg talc or silicon dioxide to the dry granule.

A further more preferred embodiment according to the invention is related to a method for preparation of a formulation according to the invention comprising the steps:
A-1) Providing 0.1 wt% to 80 wt% (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0.1 wt% to 40 wt% L-hydroxypropyl cellulose and/or povidone K25 and 2 wt% to 15 wt% sodium croscarmellose are sieved in a dry state preferably separately.
A-3) adding a solvent which leads to a particle agglomeration and the formation of the granulate structure,
A-4) sieving the granulate mass of step A-3) in a wet state,
A-5) drying the granule mass in a wet state, and
B-1) adding 5 wt% to 75 wt% adipic acid and 0.1 wt% to 10 wt% talc or silicon dioxide to the dry granule.

In a step B-1'), the excipient acidifier and/or lubricant/glidant preferably can be sieved preferably separately if two excipients are sieved.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, a polymeric precipitation inhibitor and a disintegrant in a dry state,
A-3) adding a solvent which leads to a particle agglomeration and the formation of the granulate structure,
A-4) sieving the granulate mass of step A-3) in a wet state,
A-5) drying the granule mass in a wet state,
B-1') sieving an acidfier and a lubricant/glidant such as talc or silicon dioxide, and
B-1) adding the sieved acidifier and lubricant/glidant to the dry granulate.

A preferred embodiment according to the invention is related to a method for preparation of a formulation according to the invention comprising the steps:
A-1) Providing compound 1 mg to 500 mg (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 10 mg to 50 mg polymeric precipitation inhibitor and 10 mg to 40 mg disintegrant in a dry state,
A-3) adding a solvent which leads to a particle agglomeration and the formation of the granulate structure,
A-4) sieving the granulate mass of step A-3) in a wet state,
A-5) drying the granule mass in a wet state,
B-1') sieving 10 to 360 mg acidifier and 1 mg to 20 mg lubricant/glidant such as talc or silicon dioxide, and
B-1) adding the sieved acidifier and lubricant/glidant to the dry granulate.

Another preferred embodiment according to the invention is related to a method for preparation of a formulation according to the invention comprising the steps:
A-1) Providing compound 0.1 wt% to 80 wt% (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, 0.1 wt% to 40 wt% polymeric precipitation inhibitor and 2 wt% to 15 wt% disintegrant in a dry state,
A-3) adding a solvent which leads to a particle agglomeration and the formation of the granulate structure,
A-4) sieving the granulate mass of step A-3) in a wet state,
A-5) drying the granule mass in a wet state,
B-1') sieving 1 wt% to 75 wt% acidifier and 0.1 wt% to 10 wt% lubricant/glidant such as talc or silicon dioxide, and
B-1) adding the sieved acidifier and lubricant/glidant to the dry granulate.

In a step C-1), the formulation in different dosage forms can be obtained by filling the powder mixture of step B-1) in hard gelatine capsule or by pressing the powder mixture or granule to a tablet. The powder mixture or granules are already dosage forms. In step, C-1), also a solution suitable for a parententeral admistration e.g. intravenous can be achieved if a solvent is added in step B-1) as an excipient. Also a formulation for the parenteral administration in solid form which is used for the preparation of solution before the adminstration is conceivable.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate,
B-1) adding an excipient, and
C-1) obtaining the systemic formlulation.

Thus, an embodiment according to the invention is related to a method for preparation of a formulation according to the invention comprising the steps:
A-1) Providing (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, and
A-2') sieving the (S,E)-methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate or an enantiomer, a solvate, hydrate or a pharmaceutically acceptable salt thereof, a polymeric precipitation inhibitor and a disintegrant in a dry state,
A-3) adding a solvent which leads to a particle agglomeration and the formation of the granulate structure,
A-4) sieving the granulate mass of step A-3) in a wet state
A-5) drying the granule mass in a wet state,
B-1) adding adipic acid and talc to the dry granulate, and
C-1) obtaining the systemic formulation by filling the powder mixture of step B-1) in hard gelatine capsule or by pressing the granule or powder mixture to a tablet.

### Description of the Figures:

- Figure 1: shows (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate (ZED1227) in the UUO-model.
- Figure 2: shows immune-histochemical evidence of the complex of (S,E)-methyl-7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate (ZED1227) and TG2 in the kidney tissue at different dosages.
- Figure 3: shows (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate (ZED1227) in the NASH-model.
- Figure 4: shows the pharmacokinetic of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate in the C57BL/6-mouse.
- Figure 5: shows the dose-activity relationship. For the STAM- and UUO-model the anti-fibrotic effect in dependence of the dose or in dependence of the exposition (Plasma Cmax or Plasma-AUC) are depicted.
- Figure 6: shows the plasma profiles of the compound as well as the human pharmacokinetic data derivable thereof after the multiple dosing according to example 7.
- Figure 7: shows the correlation of the humane pharmacokinetic data with the in vitro data for the TG2 inhibition.
- Figure 8: shows the maximal plasma concentration (Cmax) reached after administration of different doses of compound of formula I in formulation or as a plain compound. In order to compile the data from different species, doses were converted into human-equivalent-dose (HED) taking into account differences in the body surface area between species. The widely accepted species-specific conversion factors defined in the FDA guideline "Estimating the Maximum Safe Starting Dose in Initial Clinical Trials for Therapeutics in Adult Healthy Volunteers" (Issue date 2005) were used.
- Figure 9: shows anti-fibrotic effects in the animal model in dependence of the Cmax value as well as AUC value. In addition, the Cmax value being achieved in human by the dosage of 10, 20, and 50 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate is shown.
- Figure 10: shows the anti-fibrotic effects in the animal model in dependence of the AUC value. In addition, the AUC value being achieved in human by the dosage of 10, 20, and 50 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate is shown.
- Figure 11: shows the saturation solubility of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate.

### EXAMPLES

### Example 1

### Preparation of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate (Compound of formula (I))

### Example 1.1 Preparation of 6-amino-hept-2-en-dicarboxyl acid derivatives

### (S)-1-tert-Butyl 5-methyl 2-(tert-butoxycarbonylamino)pentanedioate

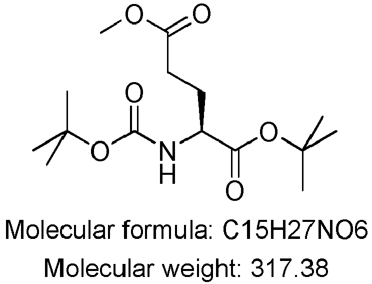

12.0 g of Boc-Glu-OtBu (39.6 mmol) are dissolved in 200 mL of DMF. Under argon atmosphere, 7.09 g of cesium carbonate (21.8 mmol, 0.55 eq.) are added and the resulting suspension is stirred for 1 hour at RT. After this time, 2.47 mL of methyl iodide (39.6 mmol) are added and stirred at RT overnight. The solvent is removed in vacuo and the obtained residue is taken up in 400 mL of ethyl acetate. The undissolved solid is filtered and the filtrate is washed with respectively 75 mL of 10% citric acid, 10% NaHCO₃ solution and brine 3 times. After drying of the organic phase over Na₂SO₄ the solvent is removed in vacuo. The product is obtained as yellow oil. The product can be used without further purification in the following reaction.
Yield: 13.4 g, >100 %
ESI-MS: 340.2 [M+Na]⁺

### (S)-1-tert-Butyl 5-methyl 2-(bis(tert-butoxycarbonyl)amino)pentanedioate

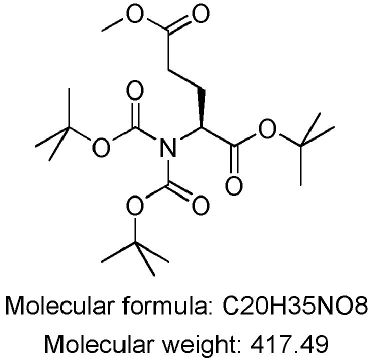

13.4 g of Boc-Glu(OMe)-OtBu (∼39.6 mmol) are dissolved in 30 mL of acetonitrile and treated with 986 mg of DMAP (7.91 mmol, 0.2 eq). Under nitrogen atmosphere a solution of 17.6 g of di-*tert*-butylbicarbonate (77.1 mmol, 2 eq) in 100 mL of acetonitrile is added. After stirring overnight, the solvent is removed in vacuo and the obtained crude product is purified by chromatography on silica gel (column: 31*6.0 cm, petroleum ether/ethyl acetate 9:1)
Column chromatography: collected in 250 mL fractions, product: fractions 6-13 TLC control: petroleum ether/ethyl acetate 8:2, R_{f} = 0.70
Yield: 13.7 g, 32.8 mmol, 83 %
ESI-MS: 440.3 [M+Na]⁺

### (S)-tert-Butyl 2-(bis(tert-butoxycarbonyl)amino)-5-oxopentanoate

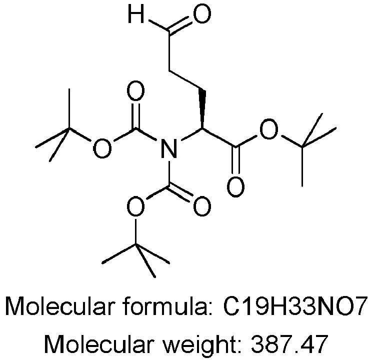

13.7 g of Boc₂-Glu(OMe)-OtBu (32.8 mmol) are dissolved in 200 mL of absolute diethylether and cooled to - 78 °C under argon atmosphere. At this temperature 36.1 mL (36.1 mmol, 1.1 eq) of a solution of diisobutyl aluminum hydride (1 M in hexane) is dropped slowly. After the addition, the solution is stirred for further 15 min at -78 °C, before the reacting mixture is quenched by addition of 50 mL of water at the same temperature. With vigorous stirring, it is warmed up to RT and the cloudy solution is filtered over Celite. The filtrate is concentrated in dryness and the residual water is removed by codestillation with toluene. Light-colored oil is obtained and it is used without further purification in the subsequent reaction. TLC control: petroleum ether/ethyl acetate 8:2, R_{f} = 0.54
Yield: 13.3 g, >100 % (purity 86.1 %)
500-MHz-¹H-NMR-cosy (DMSO_{d6}): δ[ppm] = 9.65 (s, 1H, H-4), 4.63 (dd, 1H, H-1, J_{1/2a} = 4.8 Hz, J_{1/2b} = 9.85 Hz), 2.51-2.50 (m, 1H, H-3ₐ), 2.48-4.40 (m, 1H, H-3_{b}), 2.27-2.20 (m, 1H, H-2a), 1.98-1.91 (m, 1H, H-2_{b}), 1.44 (s, 18H, 6*CH₃(Boc)), 1.92 (s, 9H, 3*CH₃(O-tBu)
ESI-MS: 410.4 [M+Na]⁺

### (S,E)-7-tert-Butyl 1-methyl 6-(bis(tert-butoxycarbonyl)amino)hept-2-enedioate

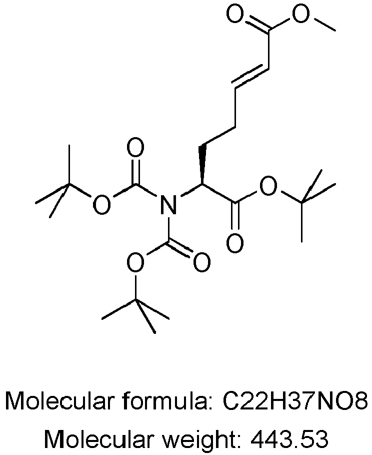

13.2 g of Boc₂-Glu(H)-OtBu (∼32.8 mmol) are provided in 20 mL of dried benzene and under argon atmosphere at RT a solution of 11.2 g of (methoxycarbonylmethylen)-triphenyl-phosphorane (32.8 mmol) is added. After stirring overnight, the solvent is removed in vacuo and the obtained oily residue is purified by chromatography on silica gel (column: 39*6.0 cm, petroleum ether/ethyl acetate 9:1).

Column chromatography: collected in 250 mL fractions, product: fractions 2-12 TLC control: petroleum ether/ethyl acetate 8:2, R_{f} = 0.54
Yield: 12.0 g, 27.1 mmol, 83 %
500-MHz-¹H-NMR-cosy (DMSO_{d6}): δ [ppm] = 6.66 (dt, 1H, H-4, J_{4/3} = 6.8 Hz J_{4/5} = 15.9 Hz), 5.64 (d, 1H, H-5, J_{5/4} = 15.9 Hz), 4.45-4.2 (m, 1H, H-1), 3.44 (s, 3H, CH₃-6), 2.01-1.95 (m, 2-H, H-3ₐ, H-3_{b}), 1.95-1.86 (m, 1H, H-2ₐ), 1.78-1.67 (m, 1H, H-2_{b}), 1.24 (s, 18H, 6*CH₃(Boc)), ESI-MS: 466.3 [M+Na]⁺

### (S,E)-2-(tert-Butoxycarbonylamino)-7-methoxy-7-oxohept-5-enoic acid (1a1)

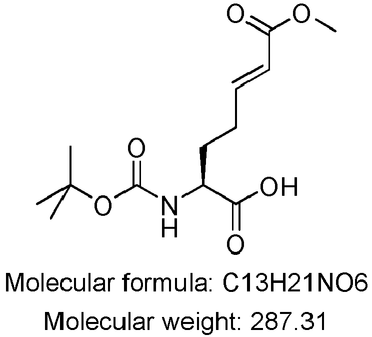

7.0 g of (S,E)-7-tert-butyl 1-methyl 6-(bis(tert-butoxycarbonyl)amino)hept-2-enedioate (15.8 mmol) are dissolve in 40 mL of dichloromethane and added into the solution of 70 mL of trifluoroacetic acid. It is stirred at RT for 4 h. The solvent is removed in vacuo and the green residue is dried under high vacuum. The obtained oil is further used without purification. By successive addition of DIPEA the pH value is adjusted to ca. 7.

The oil is taken up in 50 mL of DMF and treated with 5.37 mL of DIPEA. 4.08 g of Boc-OSu (18.9 mmol, 1.2 eq) are added and stirred at RT overnight. The solvent is removed in vacuo and the residue is suspended in 130 mL of 5% KHSO₄ solution. It is extracted with ethyl acetate (1 x 150 mL, 2 x 100 mL) and the corrected organic phases are washed with brine (75 mL). After drying of the organic phase over Na₂SO₄ the solvent is removed in vacuo. The residue is purified by chromatography on silica gel (column: 13*6.0 cm, toluene/ethyl acetate 65:35, 0.5 % acetic acid). Colorless oil is obtained.
Column chromatography: collected in 200 mL fractions, product: fractions 2-5, first running 500 mL
TLC control: toluene/ethyl acetate 1:1, 0.5% acetic acid, R_{f} = 0.35
Yield: 4.04 g, 14.1 mmol, 89 % (purity 88.6 %); ESI-MS: 310.1 [M+Na]⁺

### Example 1.2 Preparation of pyridinone derivatives

### Benzyl-3-hydroxypyridin-3-yl-carbamate

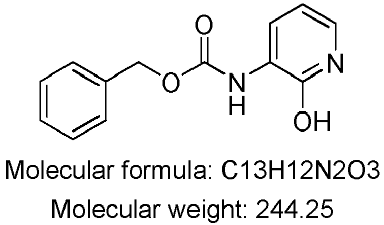

15 g of 2-hydroxy-nicotinic acid (108 mmol) are suspended in 180 mL of dried dioxane. After addition of 14.9 mL of triethylamine (108 mmol), the suspension is clear extensively. 24 mL of diphenyl phosphoryl azide (DPPA, 108 mmol) are added and the reaction solution is refluxed (130 °C) under argon atmosphere. Thereby, a gas emission is observed. After 16 h, further 16.3 mL of TEA and 12.8 mL of benzyl alcohol (117 mmol, 1.1 eq) are added successively and refluxed for further 24 h.

The solvent is removed in vacuo and the obtained brown residue is taken up in a mixture of 300 mL of DCM and 300 mL of brine. By 1M HCl solution the pH value is adjusted to ca. 1 (ca. 22 mL), the phases are separated and subsequently the water phase is extracted two times with each 200 mL of DCM. The corrected organic phases are washed with 10 % NaHCO₃ solution (3x150 mL) and brine (1 x 150 mL), dried over Na₂SO₄, filtered and concentrated in vacuo in dryness. The obtained brown solid is recrystallized from 300 mL of methanol.
TLC control: DCM/MeOH 9:1, Rf = 0.70
Yield: 16.2 g, 66.4 mmol, 62 % (pale brown, felt-like solid)
ESI-MS: 245.1 [M+H]⁺

### tert-Butyl 2-(3-(benzyloxycarbonylamino)-2-oxopyridin-1(2H)-yl)acetate

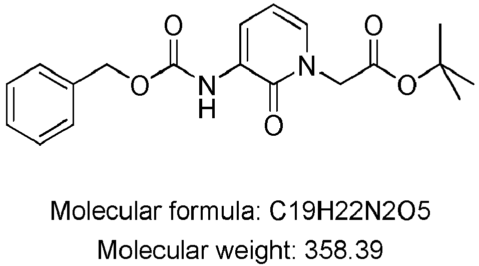

16.2 g of benzyl-3-hydroxypyridin-3-yl-carbamate (66.4 mmol) are suspended in 900 mL of absolute THF and cooled to 0°C under argon atmosphere and 2.92 g of NaH (60 % in mineral oil, 73.1 mmol, 1.1 eq) are added. To the resulting solution after the end of gas emission (ca. 15 min) 13.7 mL of bromoacetic acid tert-butylester (89.7 mmol, 1.35 eq) are added. It is stirred still for 15 minutes at 0 °C and subsequently at RT overnight. The reaction mixture is filtered and the filtrate is concentrated in dryness. The residue is taken up in 5 mL of ethyl acetate and treated with ca. 50 mL of diethylether and the resulting suspension is precipitated in the refrigerator overnight. The crystals are filtered off and washed with a little amount of ether.

The filtrate is concentrated and purified by chromatography on silica gel. (bed: 20 x 6 cm, eluent: petroleum ether/ethyl acetate = 8/2)
Column chromatography: collected in 250 mL fractions, product: fractions 10-25
TLC control: petroleum ether/ethyl acetate = 7/3, Rf = 0.46
Yield: 19.3 g, 54.0 mmol, 81 %
ESI-MS: 359.1 [M+H]⁺

### 2-(3-(Benzyloxycarbonylamino)-2-oxopyridin-1(2H)-yl)acetic acid

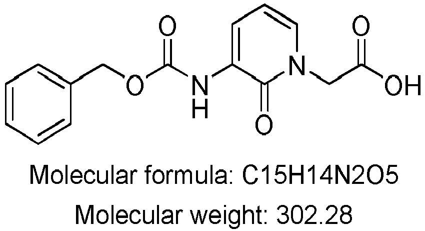

4.00 g of tert-butyl 2-(3-(benzyloxycarbonylamino)-2-oxopyridin-1(2H)-yl)acetate (11.2 mmol) are dissolved in 50 mL of dichloromethane and treated with 50 mL of trifloroacetic acid. It is stirred at RT for 3 h, before the volatile components are removed in vacuo. After drying under high vacuum a brown solid is obtained and it is suitable for the further use without purification.
Yield: 3.70 g, >100 %
ESI-MS: 303.2 [M+H]⁺

### Benzyl-1-(2-(2-ethylbutylamino)-2-oxoethyl)-2-oxo-1,2-dihydro-pyridin-3-ylcarbamate

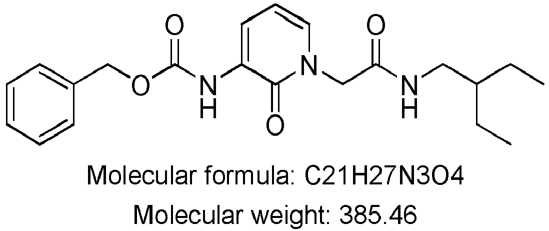

A mixture of 3.70 g of 2-(3-(benzyloxycarbonylamino)-2-oxopyridin-1(2H)-yl)acetic acid (∼11.2 mmol), 3.58 g of TBTU (11.2 mmol), 1.51 g of HOBt (11.2 mmol) is dissolved in 60 mL of DMF. By addition of 5.70 mL of DIPEA (33.5 mmol, 3 eq) a pH value is adjusted to ∼10. 1.50 mL of 2-ethyl-butylamine (11.2 mmol) is added and the mixture is stirred at RT overnight. The solvent is removed in vacuo and the obtained residue is taken up in 300 mL of DCM and subsequently washed with 10% citric acid (3x75 mL), saturated NaHCO₃ solution (3 x 75 mL) and brine (75 mL). The organic phase is dried over Na₂SO₄, filtered and concentrated in dryness. Pale brown solid is obtained and it is suitable for further processing without further purification.
Yield: 5.22 g, >100 %
ESI-MS: 386.3 [M+H]⁺

### 2-(3-Amino-2-oxopyridin-1(2H)-yl)-N-(2-ethylbutyl)acetamide (2a)

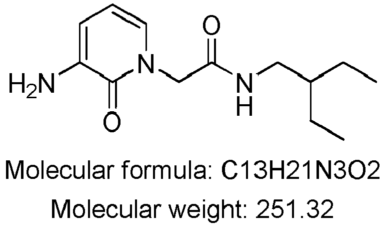

5.22 g of benzyl-1-(2-(2-ethylbutylamino)-2-oxoethyl)-2-oxo-1,2-dihydro-pyridin-3-ylcarbamate (2.4, ∼11.2 mmol) are dissolved under nitrogen atmosphere in 60 mL of methanol. To this solution, 500 mg of Pd/C (10 %) are added and stirred under hydrogen atmosphere at atmosphere pressure for 2.5 h. The catalysis is separated by filtration over silica gel, before the solvent is removed in vacuo. Dark oil is obtained and it is suitable for further processing without further purification.
Yield: 3.62 g, >100 %
ESI-MS: 252.2 [M+H]⁺

### Example 1.3 Preparation of (S,E)-Methyl 6-(tert-butoxycarbonylamino)-7-(1-(2-(2-ethylbutylamino)-2-oxoethyl)-2-oxo-1,2-dihydro-pyridin-3-ylamino)-7-oxohept-2-enoate

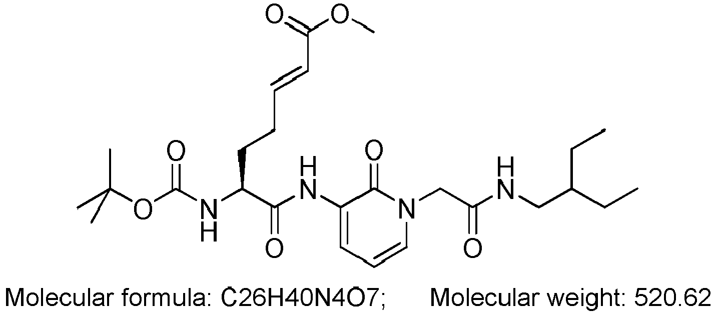

A solution of 3.36 g of 2-(3-amino-2-oxopyridin-1(2H)-yl)-N-(2-ethylbutyl)acetamide (2a, ∼10.4 mmol) in 20 mL of DMF is provided. To this solution, a solution of 2.97 g of (S,E)-2-(tert-butoxycarbonylamino)-7-ethoxy-7-oxohept-5-enoic acid (1a1, 10.4 mmol), 3.93 g of HATU (10.4 mmol) and 3.52 mL of DIPEA (20.7 mmol, 2 eq) in 40 mL of DMF is added. By successive addition of DIPEA the pH value is adjusted to ca. 7. The reaction mixture is stirred at 40 °C for 2.5 hours, as well as at RT overnight, before the solvent is removed in vacuo. The obtained brown residue is taken up in 250 mL of ethyl acetate and subsequently washed with 10 % citric acid (3x75 mL), saturated NaHCO₃ solution (3x75 mL) and brine (75 mL). The organic phase is dried over Na₂SO₄ and concentrated in vacuo in dryness. The residue is purified by chromatography on silica gel (bed: 13 x 6 cm, eluent: toluene / acetone = 7/3).
Column chromatography: 150 mL first running, corrected in 40 mL fractions, product: fraction 6-15
TLC control: DCM/MeOH = 97/3, Rf = 0.40
Yield: 3.34 g, 6.42 mmol, 62 %
ESI-MS: 543.4 [M+Na]⁺

### (S,E)-Methyl 7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate (Compound of formula (I))

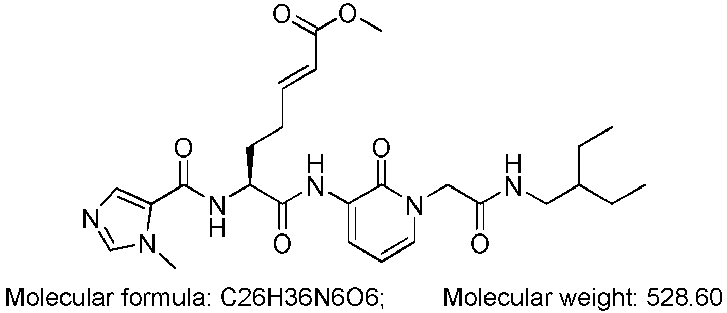

3.14 g of (S,E)-methyl 6-(tert-butoxycarbonylamino)-7-(1-(2-(2-ethylbutylamino)-2-oxoethyl)-2-oxo-1,2-dihydro-pyridin-3-ylamino)-7-oxohept-2-enoate (3.1, 6.03 mmol) are dissolved in a mixture of 25 mL of dichloromethane and 35 mL of TFA and stirred for 3 hours at RT, before the volatile components are removed in vacuo. The obtained brown oil is dried under high vacuum and dissolved in 10 mL of DMF and 1.03 mL of DIPEA (6.03 mmol) is added. To this a solution of 2.29 g of HATU (6.03 mmol) and 1.03 mL of DIPEA (6.03 mmol) in 30 mL of DMF is added. By successive addition of DIPEA the pH value is adjusted to ca. 7. It is stirred overnight at RT. The residue is taken up in 200 mL of ethyl acetate and subsequently washed with 10 % citric acid, saturated NaHCO₃ solution and brine (each 75 mL). The organic phase is dried over Na₂SO₄, filtered and concentrated in vacuo in dryness. The residue is purified by chromatography on silica gel (bed: 12 x 6 cm, eluent: DCM/MeOH = 97/3, after 2 Liters 95/5).
Column chromatography: 1000 mL first running, corrected in 50 mL fractions, product: fraction 43-66
TLC control: DCM/MeOH = 97/3, R_{f} = 0.30
Yield: 1.42 g, 2.69 mmol, 45 %
ESI-MS: 551.3 [M+Na]⁺
¹H-NMR (DMSO-d6, 500 MHz): δ [ppm] = 9.29 (s, 1H), 8.63 (d, 1H), 8.21 (dd, 1H), 8.04 (t, 1H), 7.75 (d, 2H), 7.33 (dd, 1H), 6.93 (dt, 1H, J = 15.63; 6.93), 6.25 (t, 1H), 5.86 (d, 1H, J = 15.69), 4.58 (s, 2H), 3.79 (s, 3H), 3.62 (s, 3H), 3,01 (t, 2H), 2.33 (m, 2H), 2.03 (m, 1H), 1.90 (m, 1H), 1.26 (m, 5H), 0.83 (t, 6H)

### Example 2

### Study in a unilateral ureteral obstruction model (UUO-model) for nephritic fibrosis

The anti-fibrotic effect of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate (compound of formula (**I**)) in the kidney was investigated in an UUO-model in the C57BL/6-mouse. In a UUO-model, in one of the kidneys fibrosis is induced by ligation of one ureter. The other kidney remains functional. The pathophysiology of the UUO-model resembles the chronic nephropathy in humane (Chronic Kidney Disease, CKD).
Groups of 8 UUO-mice were treated directly after ligation of the ureter over a time period of two weeks with three different dosages of said compound: 100 mg/kg body weight, 300 mg/kg body weight, and 1000 mg/kg body weight. The daily dosage was administered orally as a suspension at intervals of 8 hours. In a control group, the UUO-mice were treated in same way but merely with the vehicle methyl cellulose. After the end of the treatment, the histopathological investigation of the kidney tissue as well as the determination of the amount of hydroxyproline in the kidney was performed. Furthermore, an immune-histochemical localisation of the complex of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate and TG2 by means of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate-specific antibody was carried out.

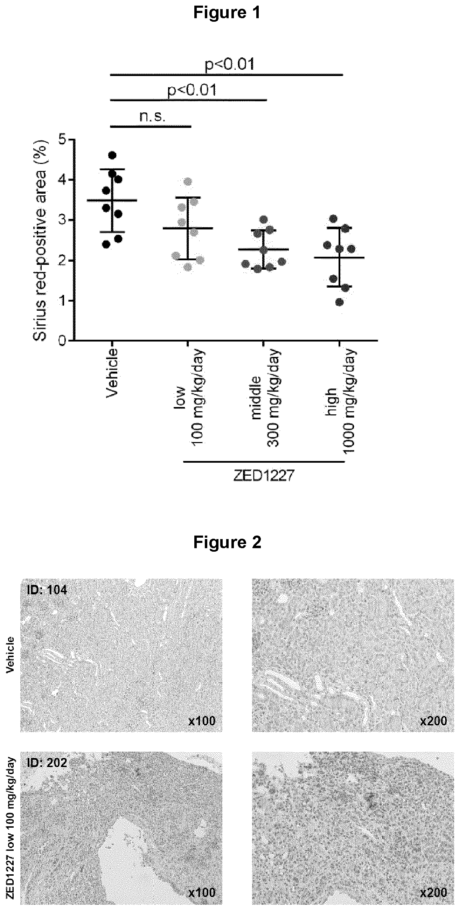

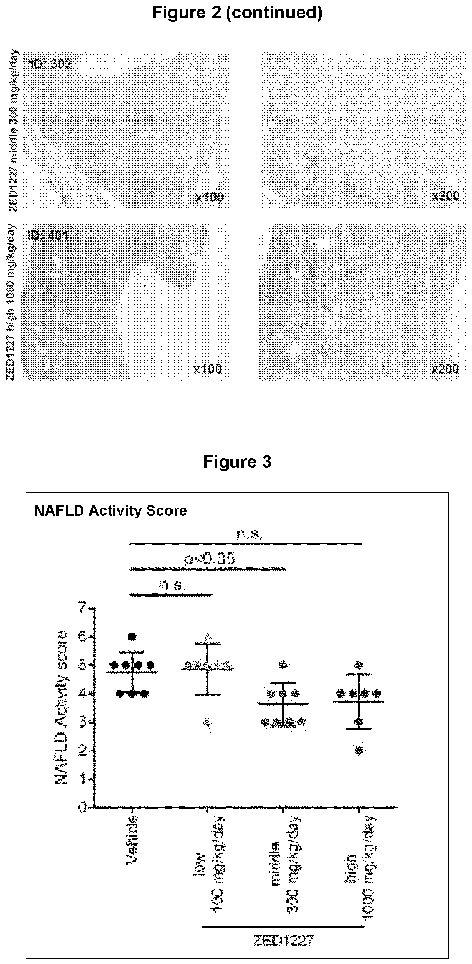

Results: The treatment with (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate results in a significant, marked, dosage dependent reduction of the fibrotic area of the kidney. The hydroxyproline content remained unaffected. The effect of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate in the UUO-model is shown in Figure 1. The immune-histochemical localisation of the complex of compound of formula (**I**) and TG2 in the kidney tissue at different dosages can be seen in Figure 2.

Conclusion: The remarkable improvement of the kidney histology in comparison to the control shows that the compound of the formula (I) supressed both fibrosis and fibrogenesis. The effects and underlying mechanism in the kidney are possibly analog to the antifibrotic effects and mechanism in the liver.
The immune-histochemical localisation of the complex of comound of formula (I) and TG2 shows that (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate reach the target site and inhibits the target molecule.

### Example 3

### Study in the stelic animal model (STAM-model) for non-alcoholic steatohepatitis

The anti-fibrotic effect of compound of formula (**I**) in the liver was investigated in a STAM-model for non-alcholic steatohepatitis. This model bases on the induction of a chronical fibrotic inflammation of the liver by means of the combination of a chemical noxin (Streptozotocin) and a fat-containing diet in the C57BL/6-mouse. The pathology is similar to the described NASH in human.

Groups of 8 STAM-mice were treated directly over a time period of two weeks with three different dosages of said compound: 100 mg/kg body weight, 300 mg/kg body weight, and 1000 mg/kg body weight. The daily dosage was administered orally as a suspension in two parts at intervals of 8 hours. In a control group, the STAM-mice were treated in same way but merely with the vehicle methyl cellulose. After the end of the treatment, a measurement of the biochemical parameter in the Plasma and after dissection of the animals a histopathological investigation fo the liver, the determination of the fat content of the liver as well as the determination of the expression of inflammation and fibrosis marker was performed.

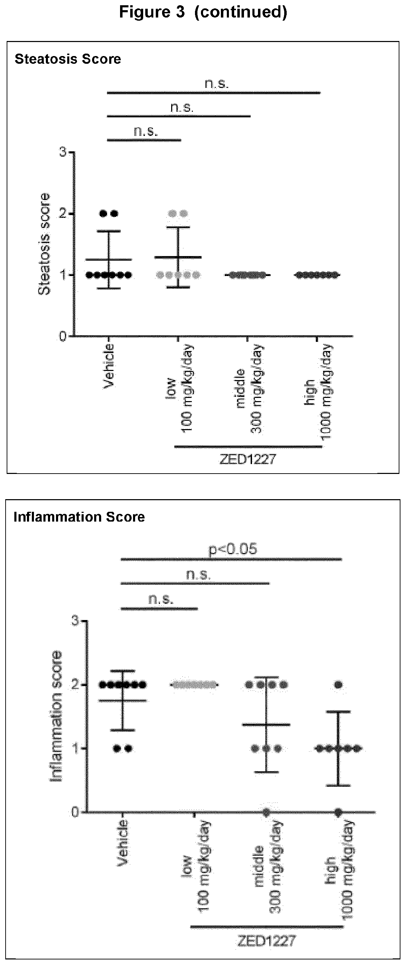

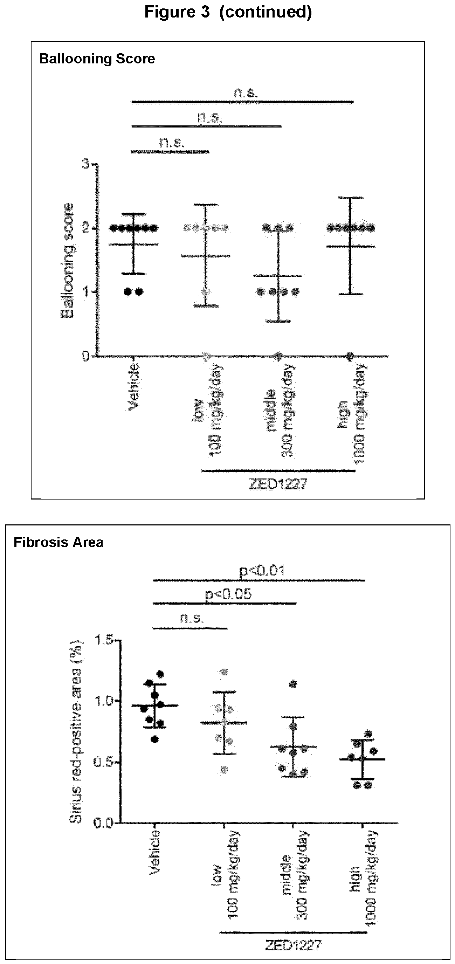

Results: The treatment with compound of formula (**I**) results in a significant, dosage dependent improvement of the liver histology. In detail, the NAFLD Activity Score (NAS) as shown in Figure 3 was reduced in the middle and high dosage group. The improvement of the NAS was mainly due to reduction of the inflammation and ballooning (inflate) of the hepatocytes. Furthermore, a marked, dosage dependent reduction of the fibrotic area in the liver could be observed which was significant in the middle and high dosage group. In comparison with historical data, the reduction of the fibrosis in the high dosage group lay in the area of Telmisartan which serves as a positive control.

Conclusion: The anti-fibrotic effect of compound of formula (**I**) is probably due to the inhibition of TG2 in the liver. As a result, the cross linking of the collagen fibrils is prevented, and the formation of fibrosis in comparision to the untreated control animals is reduced. The reduction of the inflammation and the ballooning are probably secondary to the antifibrotic effect. (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate has no influence on the fat content in the liver.

### Example 4

### Pharmacokinetic of compound of formula (I) in the C57BL/6-mouse

The study serves to describe the pharmacokinetics of compound of formula (**I**) in the C57BL/6-mouse. The dosage was analogous to the dosage in the STAM-model (example 3) or in the UUO-model (example 2) in order to ensure the estimation of the systemic exposition in these models and to correlate the pharmacokinetic data with the efficacy data.
Groups of 12 C57BL/6-mice were treated with three different dosages of compound of formula (I) over a time period of 7 days: 100 mg/kg body weight, 300 mg/kg body weight, and 1000 mg/kg body weight. The daily dosage was administered orally as a suspension in two parts at intervals of 8 hours. On day 7 of treatment three different blood samples per animal were collected. The time points in the group were distributed in such a way that the 24h-profile was covered with in total 12 different time points (3 animals per time point).

The determination of compound of formula (I) in the blood plasma was performed by means of a previously validated HLPC-MS/MS method.

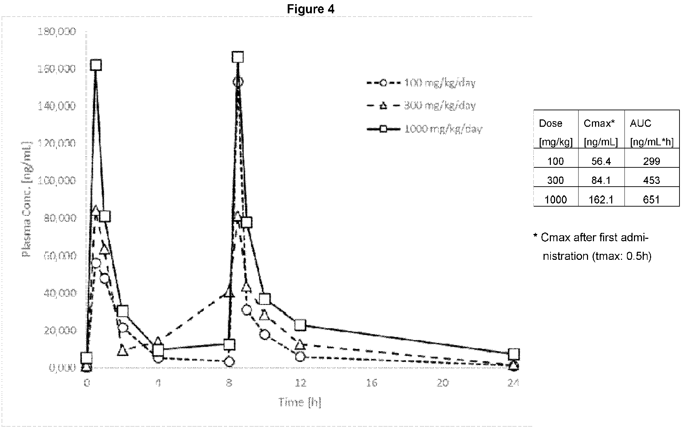

Conclusion: The plasma profile of compound of formula (**I**) as well as the pharmacokinetic data derivable thereof are depicted in Figure 4.

### Example 5

### Dose-activity-relationship

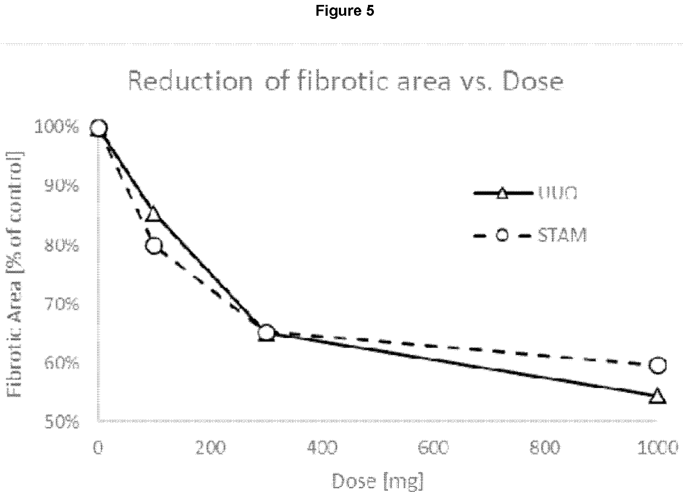

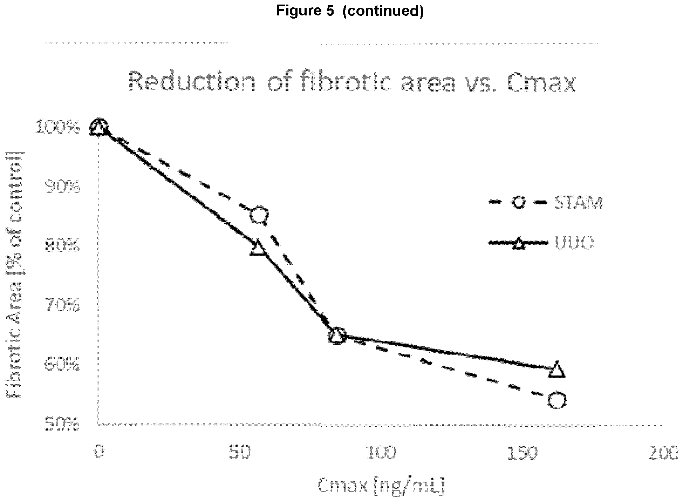

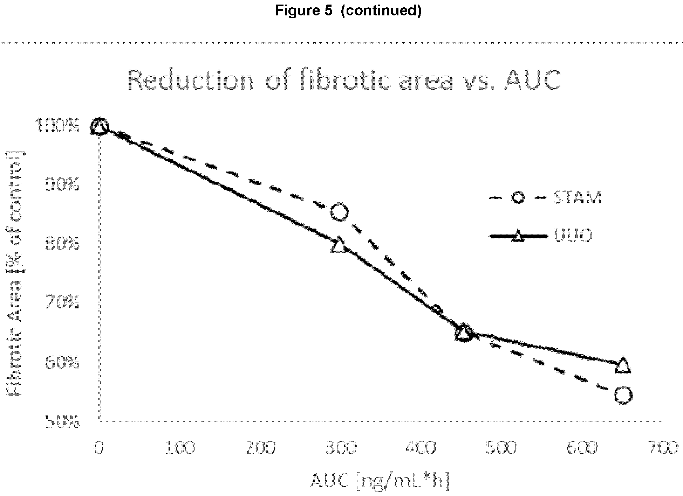

The reduction of the fibrotic area in the tissue in comparison to the untreated control animals can be considered as a measure for the anti-fibrotic effect of compound of formula (**I**) in STAM-model as well as in the UUO-model. For the STAM- and UUO-model the anti-fibrotic effect in dependence of the dose or in dependence of the exposure (Plasma Cmax or Plasma-AUC) are depicted in Figure 5.

Results: The results of the investigation of dose-activity-relationship is depicted in Figure 5.

Conclusion: Although the tissue structure of liver and kidney is different, and the pathogenesis of the fibrotic changes in the STAM-model and in the UUO-model are fundamentally different, compound of formula (I) exhibited in both models the same anti-fibrotic effect. The apparent non-linear dose-activity relationship is probably due to a saturation of the absorption and systemic exposition after the oral administration of the high dosage applied here. If the anti-fibrotic effect in dependence of the exposure in plasma is considered, an almost linear correlation is obtained.

### Example 6

### Preparation of the hard gelatine capsule

The preparation of the acidic granulate was performed by means of a wet granulation using 96% ethanol as a granulation liquid. Compound of formula (**I**), L-hydroxypropyl cellulose and sodium croscarmellose are sieved in the dry form, and mixed after that. By adding ethanol, particle agglomeration and formation of the granulate structure results. Granulate mass is sieved in wet form, dried at 70 °C, and finally sieved again. In a dry mixer, the sieved adipic acid as well as talc are added to the dry granulate and mixed. After that, the powder mixture is filled in a hard gelatine capsule.

### Example 7

### Pharmacokinetic of compound of formula (I) in human

The pharmacokinetic of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate (compound of formula (**I**)) was investigated in healthy volunteers. Cohorts of 18 subjects each were treated with a daily dose of 10, 20, 50 and 100 mg compound of formula (**I**), respectively over a time period of 7 days. The determination of said compound in blood plasma was performed by means of a previously validated HPLC-MS/MS method.

Results: The plasma profiles of the compound as well as the human pharmacokinetic data derivable thereof after the multiple dosing are depicted in Figure 6.

Conclusion: The drug concentration achieved with the formulation increases in the dose range 10 - 100 mg dose-proportionally, and when normalized to the body weight lies remarkably higher than in the animal studies in which the drug was administered as a suspension.

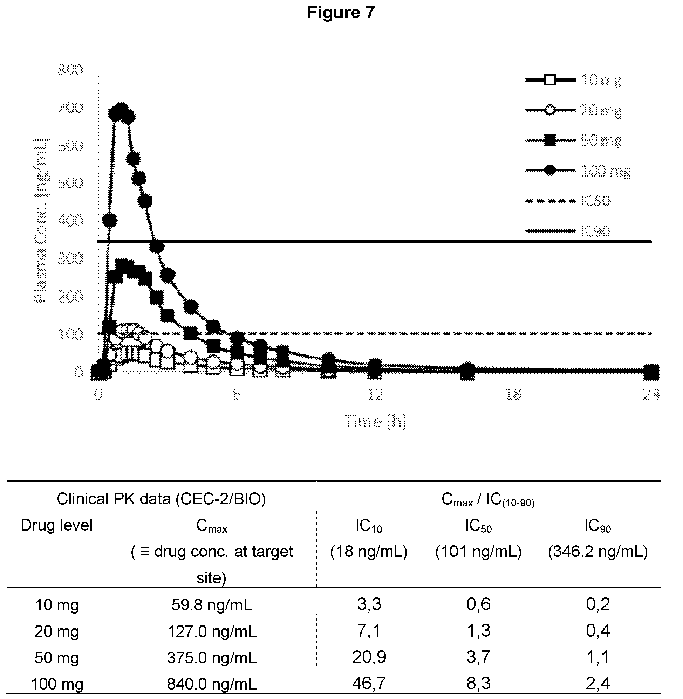

The drug concentration achieved are compared with the concentrations which in vitro result in an inhibition of the enzyme activity of TG2 (Figure 7). Thereby, the TG2-catalysed incorporation of dansyl-cadaverine into N,Ndimethylcasein (DCC-Assay), representing the cross-linking function of TG2, serves as a marker reaction. According to this correlation, the half maximal inhibition (IC₅₀) of TG2 is already achieved with a dose of 20 mg. A dose of 50 mg of the new developed formulation results in a drug level already exceeding the IC₉₀ of the enzyme inhibition. The 90% inhibition at IC₉₀ can be regarded as maximal pharmacodynamic effect.

### Example 8 Comparison of pharmacokinetic data of the formulation with pharmacokinetic data of the plain compound

The human pharmacokinetic data of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate (compound of formula (**I**)) described in example 7 were generated after administration of the hard gelatine capsule formulation described in example 6.

To estimate the influence of the formulation, the pharmacokinetic data of the hard gelatine formulation were compared with pharmacokinetic data generated after oral administration of the plain, unformulated compound. Due to regulatory and ethical reasons pharmacokinetic data with the plain compound were only generated in animals but not in human subjects.

The pharmacokinetic data of the plain compound were derived from several studies in monkeys, pigs, rabbits, rats and mice. In all studies the compound was administered orally after suspension in 0.5 % (w/v) methylcellulose in water, pH 5 ± 0.5. Blood samples were taken at various time points within 24h after drug administration and quantified by means of a previously validated HPLC-MS/MS method.

Figure 8 shows the maximal plasma concentration (Cmax) reached after administration of different doses of compound of formula I in formulation or as a plain compound. In order to compile the data from different species, doses were converted into human-equivalent-dose (HED) taking into account differences in the body surface area between species. The widely accepted species-specific conversion factors defined in the FDA guideline "Estimating the Maximum Safe Starting Dose in Initial Clinical Trials for Therapeutics in Adult Healthy Volunteers" (Issue date 2005) were used.

The comparsion in Figure 7 demonstrates over a wide dose range that the Cmax reached after administration of the formulation is more than 10-fold above the Cmax reached after administration of the plain compound. Although the differences in Cmax could partly be attributed to species differences in pharmacokinetics, the large difference between data from human and the closely related monkey suggest that the formulation plays a major role in achieving a high systemic exposure.

### Example 9

### Correlation of the anti-fibrotic effect with the human pharmacokinetic

The anti-fibrotic effect of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate in the animal model suggests a potential use of said compound in case of fibrotic liver or kidney diseases in human. A comparison of the systemic exposure (Cmax, AUC) of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate in dosage effective in the animal experiments with the systemic exposition which is achieved in human allows an estimation of the potential effective dose range.

The anti-fibrotic effects in the animal model in dependence of the Cmax value as well as AUC value are shown in Figure 9 and Figure 10. In addition, the Cmax or AUC being achieved in human by the dosage of 10, 20, and 50 mg compound of formula (I) is shown.

Conclusion: The relationship between the fibrotic area and Cmax value (Figure 9) or AUC value (Figure 10), respectively suggests that the anti-fibrotic effect shown in the animal model can already be achieved with a human dosage of 20 mg. In human, the systemic exposure further increases linearly up to 100 mg. The almost linear dependence of the anti-fibrotic effect from the systemic exposition suggests that at higher dosage a stronger anti-fibrotic effect could be achieved in human than in the animal model.

### Example 10

The saturation solubility of compound of formula (**I**) was measured in the pH-range from 1 to 6.8 using HPLC/UV.

The results are illustrated in Figure 11.

Results: It is apparent that the saturation solubility is much lower at a pH = 6.8 (small instestine) than at a pH ∼ 1.

It is remarkably that the bioavailability in the mouse model is measurable, although the saturation solubility is much lower at a pH = 6.8 than at a pH ∼ 1.

### Example 11: Preparation of a pharmaceutical composition in form of a tablet

In order to produce a solid formulation for oral administration (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate (7.6%) is mixed with L-hydroxypropyl methylcellulose (18.3%), croscarmellose sodium (1.5%), povidone K25 (8.4%) and cellulose (microcrystalline; 36.6%). The powder blend is granulated with ethanol (96%). After wet sieving the granules are dried. Adipic acid (13.7%), croscarmellose sodium (12.2%) and silicon dioxide (1.5%) are added to the granules to obtain the final blend which is compressed to tablets.

The tablet can then be coated with a film consisting of: lactose monohydrate, hydroxypropyl methylcelluose (E464; also known as hypromellose), titanium dioxide (E171), triacetin (E1518), iron oxide yellow (E172), and carnauba wax (E903).

### Example 12: Preparation of granules for capsules

50 mg L-hydroxypropyl cellulose, and 25 mg sodium croscarmellose are added to 10 mg (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate. The compounds are sieved in a dry state. 50 mg ethanol are added, whereby an granule mass is formed. The mass is sieved in a wet state, and then dried. After drying, the mass is sieved again. 115 mg adipic acid, and 12 mg talc is added to the dried granules in a in a dry mixer. Thereafter, the powder mixture is filled in a capsule.

### Example 13: Micronization process

The (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate is micronized, wherein the particle size distribution is preferably defined by d(0.1) from 0.1 to 5 µm, d(0.5) from 0.3 to 10 µm, d(0.95) from 3 to 25 µm,.,, and the particle size range is from 0.1 to 100 µm

## Claims

1. A systemic formulation containing (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate of the formula (**I**): 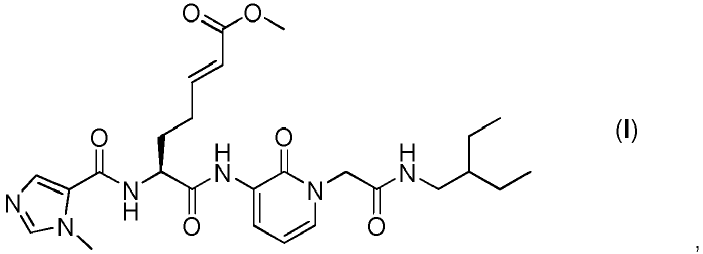 or an enantiomer, a solvate, hydrate or a pharmaceutically acceptable salt thereof.

2. The systemic formulation according to claim 1, wherein the systemic formulation is an oral formulation.

3. The systemic formulation according to claim 1 or 2, wherein the systemic formulation further comprises at least one acidifier and/or at least one binder and/or at least one polymeric precipitation inhibitor.

4. The systemic formulation according to claim 3, wherein the at least one polymeric precipitation inhibitor is selected from the group consisting of L-hydroxypropyl cellulose, hydroxypropyl cellulose, a combination of L-hydroxypropyl cellulose and hydroxypropyl cellulose, polyethylene glycol, poly(ethylene oxide)-poly (propylene oxide)-poly (ethylene oxide), polyvinyl alcohol, polyvinylpyrrolidone, carboxymethyl cellulose, methylcellulose, hydroxyethylcellulose, hydroxypropylmethylcellulose, ethylcellulose, and sodium carboxymethyl cellulose.

5. The systemic formulation according to claim 3 or 4, wherein the at least one acidifier is selected from the group consisting of ascorbic acid, organic dicarboxylic acid such as oxalic acid, malonic acid, succinic acid, glutaric acid, tartaric acid, fumaric acid, maleic acid, malic acid, adipic acid, or glutamic acid, and organic tri-carboxylic acid such as citric acid, or sodium hydrogen citrate.

6. The systemic formulation according to claim 3, 4 or 5, wherein the at least one binder is selected from the group consisting of sugar, sucrose, polysaccharides, xanthan gum, guar gum, carrageenan, starches derived from wheat, corn, rice and potatoes, preagglutinated (modified) starch derived from wheat, corn, rice and potatoes, sodium starch glycolate, natural gums, acacia gum, gelatin, tragacanth, derivatives of sea weed, alginic acid, sodium alginate, ammonium calcium alginate, cellulose, cellulose derivatives, hydroxypropyl cellulose, L-hydroxypropyl cellulose, low-substituted hydroxypropyl cellulose, methyl cellulose, sodium carboxymethylcellulose, hydroxypropyl methylcellulose, polyvinylpyrrolidone, povidone K25.

7. The systemic formulation according to any one of the claims 1 to 6, wherein (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate particles have a particle size distribution which is defined by d(0.95) ≤ 25 µm.

8. The systemic formulation according to any one of the claims 1 to 7, wherein the systemic formulation is a tablet, capsule, powder, or granule.

9. The systemic formulation according to any one of the claims 3 to 8, wherein the systemic formulation comprises 15 to 1 m/m acidifier, 0.1 to 7 m/m polymeric precipitation inhibitor, wherein m/m (mass ratio) of said compounds is calculated relative to mass of (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate.

10. The systemic formulation according to any one of claims 1 to 9, wherein the formulation comprises 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate, 3 wt% to 75 wt% acidifier, 2 wt% to 35 wt% polymeric precipitation inhibitor, 0 wt% to 12 wt% binder, 2 wt% to 35 wt% disintegrant, and 1 wt% to 9 wt% lubricant/glidant.

11. The systemic formulation according to any one of claims 1 to 10, wherein the systemic formulation comprises 0.1 wt% to 45 wt% (S,E)-methyl-7-(1-(2-(2-ethylbutylamino)-2-oxoethyl)-2-oxo-1,2-dihydro-pyridin-3-ylamino)-6-(1-methyl-1H-imidazole-5-carboxamido)-7-oxohept-2-enoate, 3 wt% to 75 wt% adipic acid, 2 wt% to 35 wt% L-hydroxypropyl cellulose and/or hydroxypropyl cellulose, 0 wt% to 12 wt% povidone K25, 2 wt% to 35 wt% sodium croscarmellose, and 1 wt% to 9 wt% talc or silicon dioxide.

12. A systemic formulation according to any one of the claims 1 to 11 for use in the prophylaxis and/or treatment of TG2-related diseases.

13. The systemic formulation according to claim 12 for use in the prophylaxis and/or treatment, wherein the TG2-related disease is selected from the group consisting of nephropathy, fibrotic liver diseases including NAFLD, NASH, cirrhosis, cholestatic liver diseases such as primary sclerosing cholangitis and primary biliary cholangitis, autoimmune hepatitis, alcoholic steatohepatitis, cystic fibrosis, pulmonary fibrosis, idiopathic pulmonary fibrosis, radiation-induced lung injury, bridging fibrosis, cardiac fibrosis, systemic sclerosis, collagen induced arthritis, rheumatoid arthritis, atrial fibrosis, endomyocardial fibrosis, old myocardial infarction, vascular stiffening, vascular calcification, fibroproliferative diseases, elevated blood pressure, gliar scar, arterial stiffness, arthrofibrosis, Dupuytren's contracture, keloid, medistinal fibrosis, myelofibrosis, Peyronie's disease, nephrongenic systemic fibrosis, progressive massive fibrosis, retroperitoneal fibrosis, systemic sclerosis, and adhesive capsulitis preferably in human.

14. The systemic formulation for use according to claim 12 or 13, wherein the TG2-related disease is selected from the group consisting of nephropathy, NASH and/or cystic fibrosis.

15. The systemic formulation for use according to any one of the claims 12 to 14, wherein the TG2-related disease is diabetes related fibrosis.
